# EUROPEAN PATENT APPLICATION

(11) **EP 2 278 636 A1**
(43) Date of publication of application: **26.01.2011**
(21) Application number: 10007514.2
(22) Date of filing: 20.07.2010
(51) Int. Cl.: H01L 51/10, H01L 51/44, H01L 51/52, H05B 33/22

(54) **Uses of dithiocarbamate compounds**

(30) Priority: 21.07.2009 EP 09009441
(71) Applicant: Sony Corporation, Tokyo 108-0075 (JP)
(72) Inventor: von Wrochem, Florian, 70327 Stuttgart (DE); Wessels, Jurina, 70327 Stuttgart (DE); Gao, Dequing, 70327 Stuttgart (DE); Ford, William, 70327 Stuttgart (DE); Rosselli, Sylvia, 70327 Stuttgart (DE); Wirtz, René, 70327 Stuttgart (DE)
(74) Representative: Appelt, Christian W.

(57) **Abstract**

The present invention relates to the use of dithiocarbamate compounds and to an assembly for use in an electronic device, said assembly comprising a self-assembled monolayer of at least one dithiocarbamate compound. The present invention also relates to an electronic device including such assembly.

## Description

The present invention relates to the use of dithiocarbamate compounds and to an assembly for use in an electronic device, said assembly comprising a self-assembled monolayer of at least one dithiocarbamate compound. The present invention also relates to an electronic device including such assembly.

Interfaces between dissimilar materials, i.e. inorganic/inorganic, organic/organic, or inorganic/organic, are inherent to most electronic devices. In organic-based electronic (including optoelectronic) devices the device performance, efficiency, and lifetime depend critically on the properties of the interfaces between the organic and inorganic components. Therefore, much effort has been directed toward modifying the inorganic/organic interface in a reliable and cost-effective manner. One of the most successful methods for doing so has been to deposit a "self-assembled monolayer" (SAM) onto one of the components, generally the inorganic one, before depositing the second component. The presence of a SAM between the organic and inorganic components of organic electronic devices can have significant effects on one or both of the components that are related to, for example, 1) a relative shift in vacuum energy level (i.e. a change in work function) or 2) a change in tunnelling barrier, both of which affect the barriers for charge transport between the components, or 3) a change in surface free energy, which affects adhesive forces between the components. SAMs have also been used for electrical passivation of semiconductor surfaces such as GaAs in junction field effect transistors. In the prior art so far, different kinds of molecules are used for SAM deposition, depending on whether the inorganic component is a conductor, a semiconductor, or an insulator. Typically, thiols are used for metals, and oxy-acid compounds (or activated derivatives thereof) are used for metal-oxide-based or metal-chalcogenide-based semiconductors or insulators. Moreover, considerable skill and effort is often required to obtain thiols or oxy-acid compounds with the desired physical chemical properties. Therefore it was an objective of the present invention to provide for a means of depositing self-assembled monolayers onto conducting, semiconducting, or insulating inorganic substrates with molecules that can be easily prepared, whose tunnelling barriers and surface free energies are easily adjusted, that have good thermal and chemical stability, and that achieve larger shifts in work function than previously achieved. All these objectives are solved by the use of a self-assembled monolayer of at least one dithiocarbamate compound.

Charge (hole or electron) injection at the interface between an electrode and an organic semiconductor material is involved in a variety of organic electronic devices, including light-emitting devices (LEDs, etc.), transistor devices (FETs, TFTs, etc.), and photovoltaic devices (solar cells, photodetectors, etc.). A Schottky barrier is present at the electrode/organic interface due to different energy level alignments of the work function of the electrode material and the highest occupied molecular orbital (HOMO) and/or the lowest unoccupied molecular orbital (LUMO) of the organic material. Hence, the Schottky barrier represents the barrier for charge injection between the two phases and its magnitude is an important factor in determining the performance, efficiency, and lifetime of virtually all organic electronic devices. The injection of electron from the surface of materials directly into vacuum under the influence of an applied electric field, known as field emission, is a process based on quantum tunneling that is used in a variety of applications, including field emission displays (FEDs).

The work function (Φ) is a fundamental property of materials that plays a key role in many physical and chemical phenomena, such as the semiconductor field-effect, photo- and thermionic electron emission, catalysis, etc. The work function is defined as the minimum work required for extracting an electron from the Fermi level of a condensed phase and placing it into the so-called vacuum level just beyond the influence of the electrostatic forces. The Fermi level of electrically conducting materials (e.g., metals or heavily doped semiconductors) is the upper limit of the valence band, while for semiconducting or insulating inorganic materials (e.g., ZnO or Al₂O₃) it is inside the band gap between the valence band and the conduction band. The work of extracting the electron from the Fermi level can be conceptually divided between the work required to free the electron from the bulk and the work associated with moving the electron through the surface.

In organic electronic devices, the dipoles formed at the electrode surface play key roles in determining the barriers for charge (hole or electron) injection from the electrodes to the active organic layers. Electrodes in general are formed from electrically conducting materials ("conductors"). For simplicity the following description refers to electrodes comprising metals, but these phenomena apply to doped semiconductors as well. The electric dipole layer that exists at a metal/molecule interface ("interface dipole") results from two contributions: one from the metal-molecule interaction and the other from the permanent (intrinsic) dipole moment of the molecule itself. The contribution from the metal-molecule interaction can also be divided into two parts, one part being induced by partial charge transfer between the molecule and the metal upon adsorption and the other part resulting from attenuation of the "tail" of the surface-localized electron wave function of the metal that "spills out" into the vacuum ("Pauli repulsion" or "Pauli exclusion"). The image potential energy is also a significant contributor to the work function shift when polarizable functional groups are attached to the molecules.

An electric dipole consists of two electric charges +*q* and -*q* are separated by a distance *d*. A molecule in which charges +*q* and -*q* are separated by a distance *d* is said to possess a "permanent dipole moment", which is referred to herein simply as the net dipole moment (µ). The charges responsible for µ could represent ionized groups within the molecule, or non-ionized but polarizing groups within the molecule, or simply an asymmetric charge distribution in a polar covalent bond. A covalent bond is considered to be "polar" when the bonding electrons are not equally shared between the two atoms, which generally is the case when the atoms have different electronegativities. A molecule having a permanent dipole moment is distinguished from one in which the dipole moment is "induced" by an external electric field. In either case, the dipole moment is a vector whose direction is given by the line connecting the centers of charge and whose magnitude is defined as µ = *qd* and whose direction is defined as going from the negative to the positive pole. The vector nature of the dipole moment means that the dipolar character of even very complex molecules can be represented as the single vector sums of all the moments of individual dipoles within the molecules. Thus the dipole moments of polyatomic molecules can be resolved into contributions from various groups of atoms within the molecules.

In recent years, self-assembled monolayers (SAMs) of organic molecules having permanent electric dipole moments ("dipolar molecules"), have been used for the purpose of tuning the interface dipole layer at electrode/organic interfaces. Molecules used for this purpose typically have a rod-like geometry and are terminated with functional groups enabling chemisorption, as described below. Self-assembly in this context refers to molecules that adsorb spontaneously onto surfaces, resulting in ordered or semi-ordered assemblies.

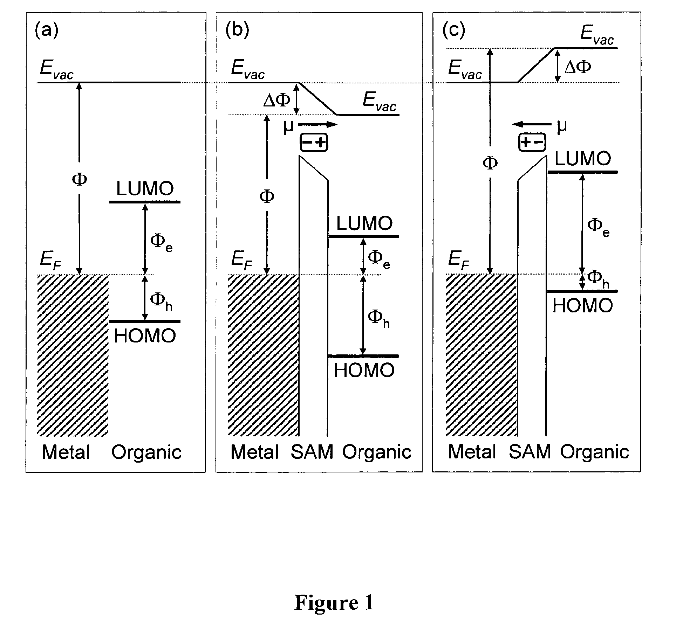

The energy level diagrams shown in Figure 1 illustrate the principle behind using self-assembled monolayers (SAMs) of dipolar molecules for modifying the work functions of metal electrodes and thus controlling charge injection barriers at metal-organic (metal-molecule) interfaces. The diagram in Figure 1a represents the interface between a metal layer on the left and and an organic layer on the right, i.e. a metal/organic interface. The Fermi energy (*E_{F}*) of the metal and the HOMO and LUMO energy levels of the organic molecule are indicated, as well as the vacuum energy level (*E_{vac}*)*.* The energy gap between *E_{F}* and *E_{vac}* represents the work function of the metal (Φ). The energy gap between *E_{F}* and the HOMO energy level of the organic molecule represents the energy barrier for hole injection from the metal (Φₕ), while the energy gap between *E_{F}* and the LUMO energy level of the molecule represents the energy barrier for electron injection from the metal (Φₑ). The other two energy level diagrams represent two possible cases when a polar SAM is sandwiched between the metal layer and the organic layer, i.e. a metal/SAM/organic interface. When the dipole µ points away from the surface of the metal, as represented in the diagram in Figure 1b, the vacuum energy level *E_{vac}* shifts downward upon crossing the SAM, resulting in a net decrease in the work function of the metal by ΔΦ; the barrier for hole injection consequently increases by ΔΦ while the barrier for electron injection decreases by ΔΦ. The opposite holds true when the dipole points towards the surface of the metal as represented in the diagram in Figure 1c, i.e. the work function of the metal increases by ΔΦ, resulting in a smaller hole injection barrier and larger electron injection barrier compared to the system with no SAM. Similar energy level diagrams apply for cases in which the electrode material is a semiconducting substance or a conducting substance other than a metal.

Generally, a layer with a net electrical dipole perpendicular to a surface can produce a substantial shift in the surface potential of any material. For a metal, this means a change in the work function (for a semiconductor it also means a change in the electron affinity and ionization potential). To a first approximation, the change in work function can be described in terms of a parallel plate capacitor if the lateral dimensions of the layer are much larger than its thickess. Based on the Helmholtz equation the potential drop ΔΦ across the dipolar layer, is given by ΔΦ = *N*µ(cosθ)/εε₀, where µ is the dipole moment, N is the dipole density per unit area, θ is the average angle between the dipole moment and the surface normal, ε is the relative permittivity of the film, and ε₀ is the permittivity of free space. The permittivity ε is introduced to take into account the polarizability of the molecules, which causes a depolarizing field at the position of a given dipole, arising from all surrounding dipoles. Thus the total change in the dipole-induced electrostatic potential depends on the molecular dipole moment as well as the surface coverage of the active molecules and their tilt relative to the surface normal. However, little is known about the "lateral" interactions of adjacent molecular dipoles, the correlation between the lateral interactions, and the electrical properties of the resulting structures.

Depolarization within a layer of atoms occurs as a result of charge transfer between the atoms and the substrate. With molecules, however, other possiblities exist including alteration of their molecular conformation resulting in changes in the distance between the two poles. However, in close-packed layers, changing the molecular conformation will normally carry too high a price in energy, not just because of the loss of van der Waals interactions but because it will require a cooperative distortion. If the molecules are very polarizable, then intramolecular charge reorganization becomes the preferred route to reduce the dipole moment. On the other hand, if the organic layer is made up of molecules with low polarizability and the molecules are close-packed in a well-ordered monolayer, then the energetically most accessible channel for reducing the field across the layer is, as for atoms, charge transfer to or from the substrate.

To first order, molecular polarizability can be taken into account using the equation u = µ₀/(1+α*k*/*a*³), where µ and µ₀ represent the molecular dipole in the array and in the gas phase, respectively, α is the molecular polarizability, *a* is the inter-dipole distance along one direction of periodicity, and k is a constant that depends only on the geometry of the periodic array. This equation reveals that a depolarization, i.e. a reduction of the molecular dipole with respect to its gas phase value is expected. Physically each molecule experiences an electric field due to all other molecules, which points in a direction opposite to that of the molecule's own dipole. Therefore, the molecule partially depolarizes in response to this on-site electric field. The potential drop across the polar monolayer is then given by the equation ΔΦ = 4πµ₀(cosθ)/[*ab*(1+α*k*/*a*³)], where *a* and *b* represent the dimensions of a rectangular unit cell, so that the product *ab* corresponds to the area per point dipole, this equation is related to the Helmholtz equation above. However, these equations only address the dielectric response of individual molecules. In densely packed molecular layers, collective effects due to intermolecular or molecule-substrate interactions, as well as significant structural changes, may take place so as to further depolarize the monolayer. Corrections have been made taking into consideration the effects of image dipoles and fluctuations in dipole orientation.

As used herein the term "monolayer" refers to a single molecular layer in which the total coverage can vary from approximately 0.7 monolayer to 1 monolayer, depending on the substituents and substrate, as opposed to a "multilayer" in which the total coverage exceeds 1 monolayer. As used herein the term "monolayer" does not distinguish between highly ordered monolayers that have both local and long-range order and less ordered monolayers that are partially organized with an average orientation perpendicular to the surface of the substrate. Ideally, the molecules comprising the monolayer are closely packed.

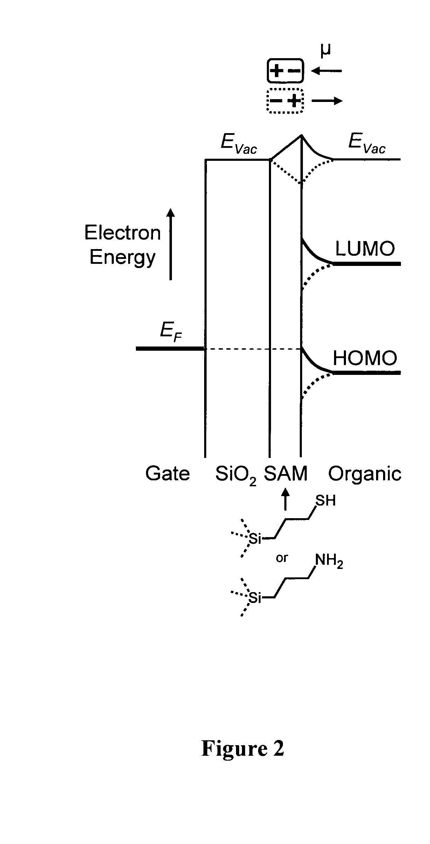

The energy level diagram shown in Figure 2 illustrates a prior art example of how self-assembled monolayers of dipolar molecules were used to modify the performance of an organic thin-film transistor (OTFT). Monolayers of various silane derivatives were chemisorbed via the silanol ((OH)₃Si-) group of the molecules onto the silicon dioxide (SiO₂) gate-insulator layer before the organic semiconductor (pentacene) was deposited (Appl. Phys. Lett. 90, 132104 (2007)), providing an inorganic(insulator)/SAM/organic(semiconductor) interface. The permanent dipole of the SAM layer influenced the work function of the insulator layer, thereby influencing band bending and doping of the semiconductor layer. The device having a SAM with an electron-withdrawing mercaptopropyl (-(CH₂)₃SH) group attached to the silicon atom had a higher field effect mobility and a lower turn-on voltage compared to the device having a SAM with an electron-donating aminopropyl (-(CH₂)₃NH₂) group attached to the silicon atom.

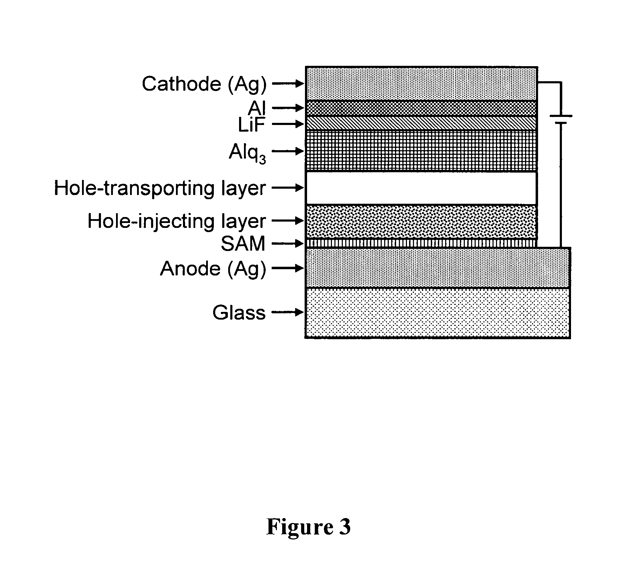

The device diagram shown in Figure 3 illustrates a prior art example of how self-assembled monolayers of dipolar molecules were used to modify the performance of a top-emitting organic light-emitting diode (TOLED) by tuning the work function of the silver (Ag) anode. Monolayers of various benzoic acid or perfluoroalkanoic derivatives were chemisorbed via the carboxylic acid (HOOC-) group of the molecules onto the anode before the organic hole-injecting layer was deposited (Langmuir 23, 7090 (2007)), providing an inorganic(conductor)/SAM/organic(semiconductor) interface. Surprisingly, the highest luminous efficiency was obtained using the molecule with the greatest tunnelling barrier for hole injection, CF₃(CF₂)₁₄COOH.

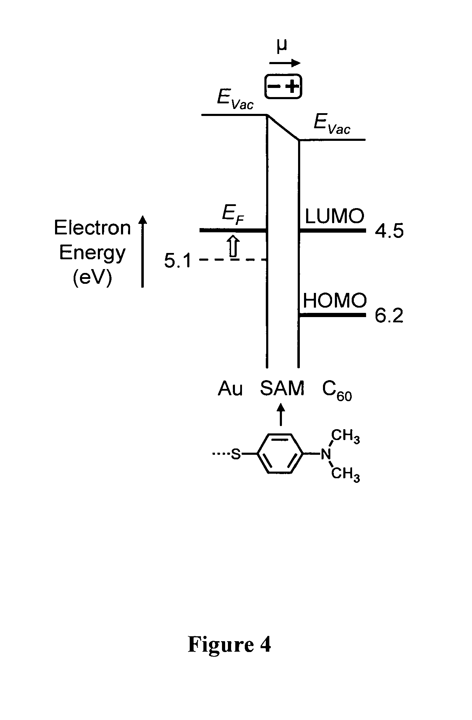

The energy level diagram shown in Figure 4 illustrates another prior art example of how self-assembled monolayers of dipolar molecules were used to modify the performance of an OTFT. Monolayers of various benzenethiol derivatives were chemisorbed via the thiol (HS-) group of the molecules onto the gold (Au) source and drain electrodes before the organic semiconductor (C₆₀) was deposited (Appl. Phys. Lett. 94, 083310 (2009)), providing an inorganic(conductor)/SAM/organic(semiconductor) interface. In the absence of a SAM, the Schottky barrier between the LUMO energy level of the C₆₀ (4.5 eV) and the Fermi energy level of the Au (5.1 eV) resulted in poor electron injection characteristics and thus poor device performance. Improved performance (ohmic contact) was achieved by using the para-substituted benzenethiol derivative with the electron-donating dimethylamino (-N(CH₃)₂) group.

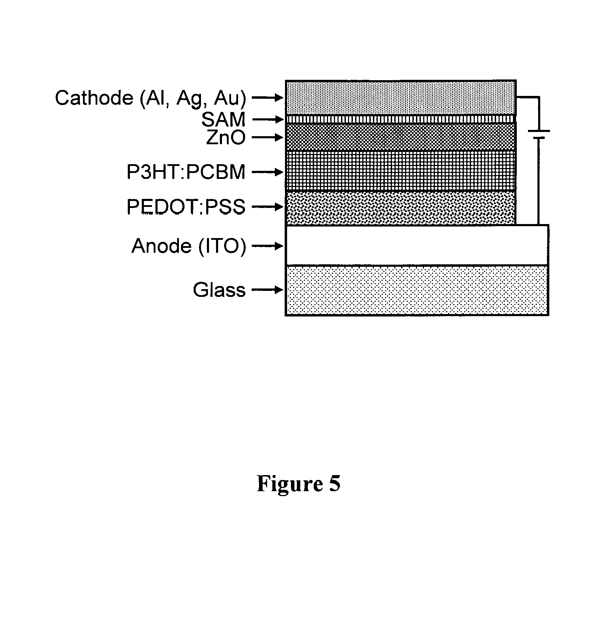

The device diagram shown in Figure 5 illustrates a prior art example of how self-assembled monolayers of various dipolar benzoic acid molecules were used to modify the performance of solid-state photovoltaic solar cells. The monolayers were chemisorbed via the carboxylic acid (HOOC-) group of the molecules onto the zinc oxide (ZnO) layer before the cathode (Al, Ag, or Au) was deposited (Adv. Mater. 20, 2376 (2008)), providing an inorganic(semiconductor)/SAM/inorganic(conductor) interface. Improved performance (increase in open-circuit voltage) was achieved by using para-substituted benzoic acid derivatives with electron-donating groups.

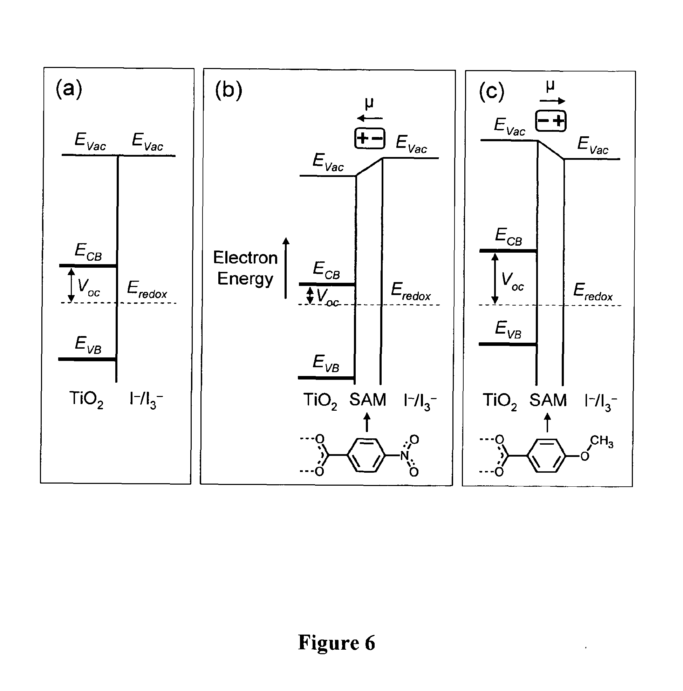

The energy level diagrams shown in Figure 6 illustrate a prior art example of how self-assembled monolayers of dipolar benzoic acid molecules were used to modify the performance of a dye-sensitized solar cell (DSSC). The monolayers were chemisorbed via the carboxylic acid (HOOC-) group of the molecules together with the dye molecules onto the titanium dioxide (TiO₂) layer before being contacted with the electrolyte solution (I⁻/I₃⁻) (J. Phys. Chem. B 109, 189074 (2005)), providing an inorganic(semiconductor)/SAM/electrolyte(conductor) interface. A decrease in the open-circuit voltage (V_{oc}) was obtained by using the para-substituted benzoic acid derivative with the electron-accepting nitro (-NO₂) group (Figure 6b), while the opposite effect was obtained by using the derivative with the electron-donating methoxy (-OCH₃) group (Figure 6c).

Various strategies have been used to design and synthesize dipolar molecules suitable for self-assembly on the surfaces of inorganic conductors, semiconductors, or insulators. To some extent, these strategies depend on the way in which the molecules are bonded to the surface. Often the bond is formed by chemisorption. Generally speaking, the adsorption of molecules at solid surfaces can occur by either chemical or physical bonding. As used herein, chemisorption (chemical adsorption) refers to the formation of bonds of chemical strength (i.e. binding energies per adsorbate in the eV range), whereas physisorption (physical adsorption) refers to unspecific adsorption based on dispersion interaction. Molecules that tend to form vertically oriented and densely packed self-assembled monolayers typically have a rod-like geometry and are terminated with functional groups enabling chemisorption, which are sometimes referred to as "anchoring groups" or "connecting groups" or "binding groups."

Based on a literature search performed by the present applicant, it appears that two trends exist: 1) the preferred anchoring group for metal electrodes is the thiol-group, and 2) the preferred anchoring groups for metal oxide semiconductor electrodes, such as ITO (indium thin oxide), TiO₂, and ZnO, are oxy-acid or oxy-acid chloride groups (e.g., HOOC-, ClOC-, Cl₃Si-, -(HO)₂OP-, Cl₂OPO-, Cl₂OS-).

Furthermore, it appears from such literature search that thiols may be used to tune the work function of gold from its intrinsic value of 5.02 eV to a value within the range of 4.0-5.7 eV. The lower values of work function are achieved by using alkane thiols such as hexadecanethiol (HS(CH₂)₁₅CH₃), while the higher values are achieved by using the fluorinated analogues (e.g., HS(CF₂)₁₅CF₃ or HS(CH₂)₁₅CF₃).

So far, different kinds of anchoring groups are required depending on whether the work functions of inorganic substances, which may be conductors, semiconductors, or insulators, are to be modified. Thus, thiols are required for metal electrodes, and oxy-acid compounds (or activated derivatives thereof) for semiconductor (metal-oxide or metal chalcogenide) electrodes. Moreover, considerable skill and effort is required to obtain thiols or oxy-acid compounds with the desired physical chemical properties. Contacts provided by thiol or oxy-acid anchoring groups have relatively high electrical resistances, which introduce barriers to electron or hole transfer across the electrode-molecule interface. Moreover, self-assembled monolayers of thiols, especially those on noble metals (e.g., Au, Ag), are unstable with respect to both thermal desorption and oxidation. Additionally, large negative shifts in work functions are generally difficult to achieve with such compounds.

Therefore it was an object of the present invention to provide for means to modify the work function of conducting, semiconducting, or insulating inorganic substrates. It was also an object of the present invention to provide for means to modify the work function of such substrates with molecules that can be easily prepared. It was also an object of the present invention to provide for means to modify the work function of such substrate with materials that have greater thermal and chemical stability than self-assembled monolayers of thiols. It was also an object of the present invention to provide for means to modify the work function of such substrates, by which means it is possible to achieve larger shifts in work function than previously achieved.

All these objects are solved by the use of a dithiocarbamate compound for modifying the work function of a conducting, semiconducting, or insulating inorganic substrate. Further advantageous features of certain embodiments of the present invention include: 1) providing a wide range of surface energies by selection of appropriate end groups on said compounds, 2) providing a wide range of tunnelling barriers by selection of non-conjugated or pi-conjugated segments within said compounds, and 3) providing a lower electrical contact resistance than is available with thiols and other groups commonly used for chemisorption.

In one embodiment said use comprises the step:
Depositing a monolayer, preferably a self-assembled monolayer, of said dithiocarbamate compound on a surface of said conducting, semiconducting, or insulating inorganic substrate.

In one embodiment said step of depositing occurs by exposing said conducting, semiconducting, or insulating inorganic substrate to a solution of a dithiocarbamate compound

In one embodiment said dithiocarbamate compound has a permanent positive or negative electrical dipole moment, preferably a dipole moment whose absolute value is equal to or greater than 4 Debye.

In one embodiment said dithiocarbamate compound has an S₂CNH- group or an S₂CNR-group in its molecular structure, wherein R denotes an alkyl, aryl, aralkyl, heteroalkyl, heteroaryl or heteroaralkyl substituent, which itself may be substituted or unsubstituted.

In one embodiment said dithiocarbamate compound is a piperazine dithiocarbamate derivative or a piperidine dithiocarbamate derivative.

In one embodiment said dithiocarbamate compound has at least one or several uncharged polar components in its molecular structure.

In one embodiment said dithiocarbamate compound has a dithiocarbamate group in its molecular structure having a dipole moment with a first polarity, and wherein said at least one or several uncharged polar components has a second polarity which is opposite said first polarity, wherein said dithiocarbamate compound is used for increasing the work function of said conducting, semiconducting, or insulating inorganic substrate.

In another embodiment said dithiocarbamate compound has a dithiocarbamate group in its molecular structure having a dipole moment with a first polarity, and wherein said at least one or several uncharged polar components has a second polarity which is the same as said first polarity, wherein said dithiocarbamate compound is used for decreasing the work function of said conducting, semiconducting, or insulating inorganic substrate.

As used herein, a dithiocarbamate compound with a positive µ (µ > 0) causes the work function of the substrate to which it is chemisorbed to decrease (ΔΦ < 0), wherein µ refers to the net dipole moment of the compound normal to the surface of the substrate when both sulfur atoms of the dithiocarbamate group are bonded to the substrate. Conversely, the work function of the substrate increases (ΔΦ > 0) when µ is negative (µ < 0).

In one embodiment said dithiocarbamate compound is selected from the group having the general structures: 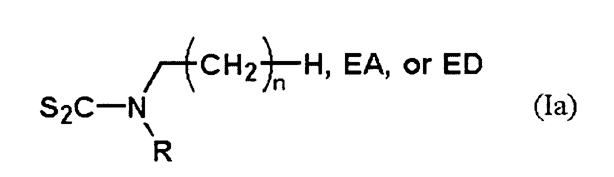 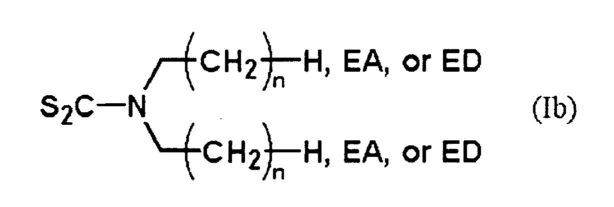 n=0-17
R = H, CH₃ or CH₂CH₃
wherein H denotes an H-atom, EA denotes an electron-accepting group, and ED denotes an electron-donating group.

In one embodiment said dithiocarbamate compound is selected from the group having the general structures: 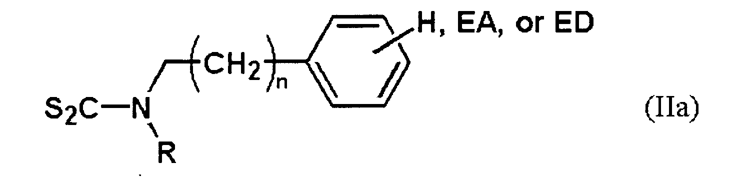 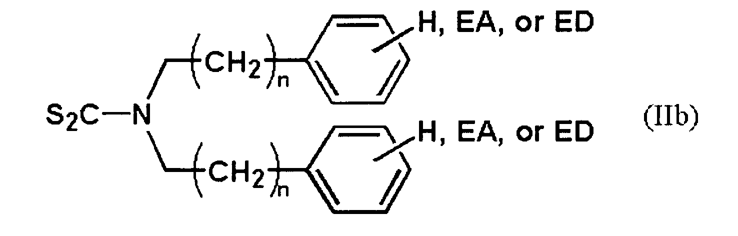 n=0-17
R = H, CH₃ or CH₂CH₃
wherein H denotes an H-atom (i.e. the benzene ring is unsubstituted except where it joins the rest of the molecule), EA denotes an electron-accepting group, and ED denotes an electron-donating group. Preferably, said EA or ED group occupies the para- and/or meta-position with respect to the bond joining the benzene ring to the rest of the molecule. More preferably, said EA or ED group occupies the para-position.

In one embodiment said dithiocarbamate compound is selected from the group having the general structures: 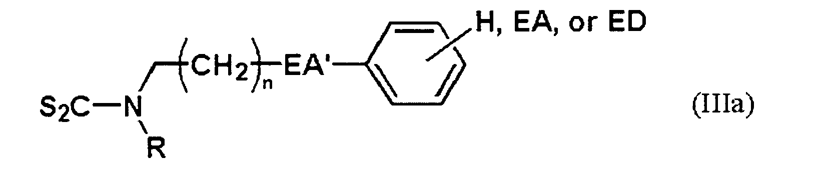 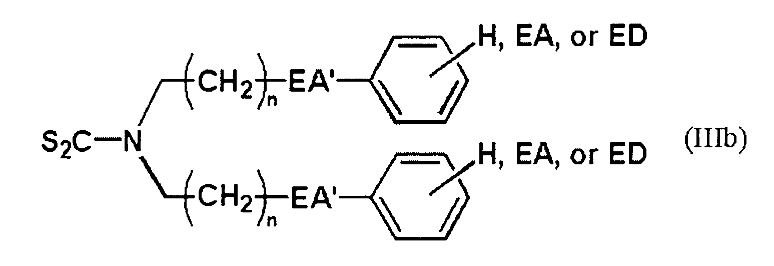 n = 0-15
R = H, CH₃ or CH₂CH₃
wherein EA' denotes a bridging electron-accepting group selected from the group comprising -OC(O)-, -C(O)O-, -C(O)-, -S(O)₂-, -N(R')C(O)-, and -N(R')S(O)₂-, where R' = H or CH₃, and wherein H denotes an H-atom (i.e. the benzene ring is unsubstituted except where it joins the rest of the molecule), EA denotes an electron-accepting group, and ED denotes an electron-donating group. Preferably, said EA or ED group occupies the para- and/or meta-position with respect to the bond joining the benzene ring to the rest of the molecule. More preferably, said EA or ED group occupies the para-position.

In one embodiment said dithiocarbamate compound is selected from the group having the general structures: 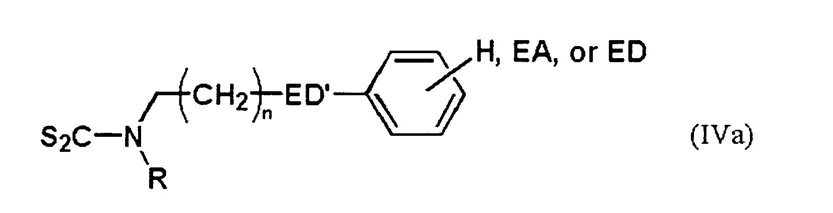 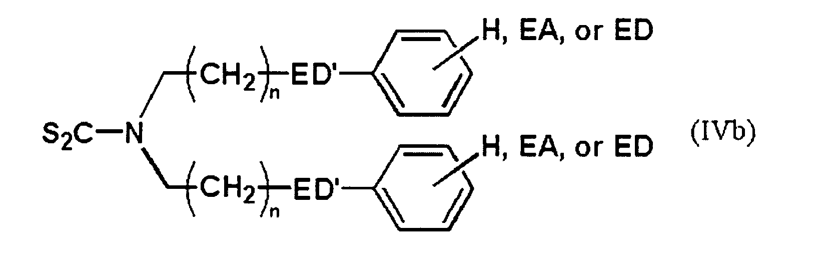 n = 0-15
R = H, CH₃ or CH₂CH₃

wherein ED' denotes a bridging electron-donating group selected from the group comprising -N(R')-, -O-, -S-, and -C(O)N(R')-, where R' = H or CH₃, and wherein H denotes an H-atom (i.e. the benzene ring is unsubstituted except where it joins the rest of the molecule), EA denotes an electron-accepting group, and ED denotes an electron-donating group. Preferably, said EA or ED group occupies the para- and/or meta-position with respect to the bond joining the benzene ring to the rest of the molecule. More preferably, said EA or ED group occupies the para-position.

In one embodiment said dithiocarbamate compound is selected from the group comprising the piperazine (X = N) or piperidine (X = CH) derivatives having the general structures:

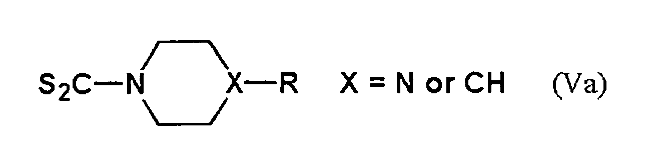

-R =

CH

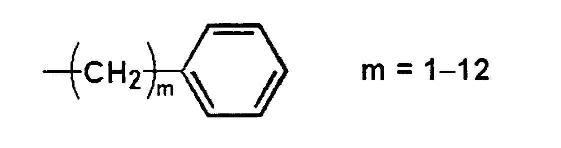

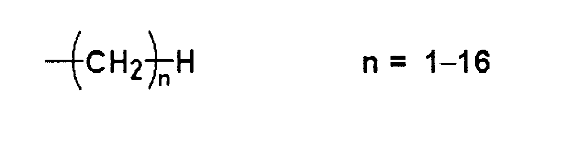

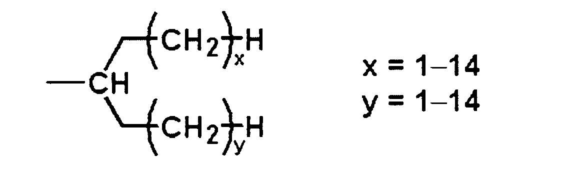

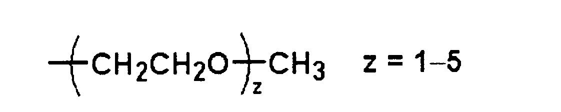

The net dipole moment (µ) in this series of compounds is mainly due to the intrinsic polarity of the dithiocarbamate (S₂CNR-) group, whose dipole is generally directed away from the surface of the inorganic substrate with a value µ ≈ +4.4 D, thereby providing a negative shift in the work function of the substrate. Inclusion of a phenyl ring or substitution of methylene groups with oxygen atoms within the σ-group reduces the tunnelling barrier and/or surface energy of the resulting SAM.

In one embodiment said dithiocarbamate compound is selected from the group comprising the piperazine (X = N) or piperidine (X = CH) derivatives having the general structures: 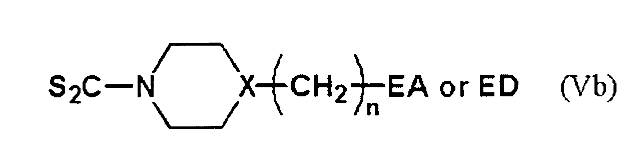 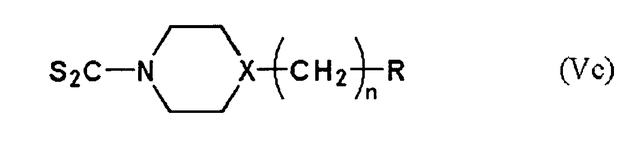 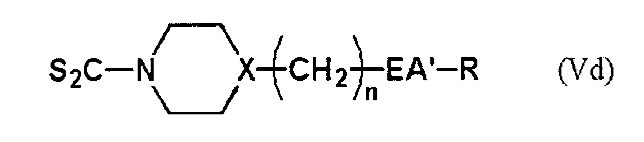 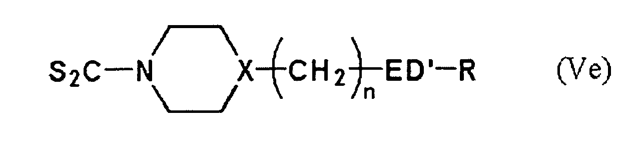 X=N or CH

n = 1-16 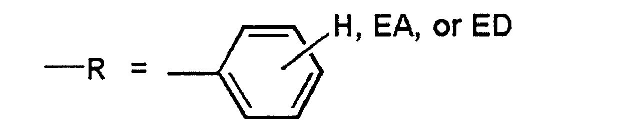 wherein EA' denotes a bridging electron-accepting group selected from the group comprising -OC(O)-, -C(O)O-, -C(O)-, and -S(O)₂-, wherein ED' denotes a bridging electron-donating group selected from the group comprising -N(R')-, -O-, and -S-, and wherein H denotes an H-atom (i.e. the benzene ring is unsubstituted except where it joins the rest of the molecule), EA denotes an electron-accepting group, and ED denotes an electron-donating group. Preferably, said EA or ED group occupies the para- and/or meta-position with respect to the bond joining the benzene ring to the rest of the molecule. More preferably, said EA or ED group occupies the para-position.

In one embodiment said dithiocarbamate compound is selected from the group comprising the piperazine or piperidine derivatives having the general structures: 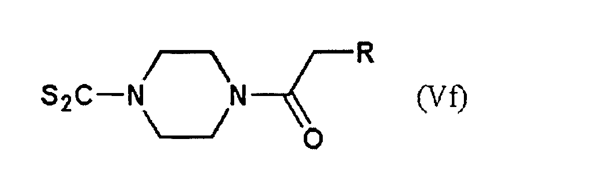 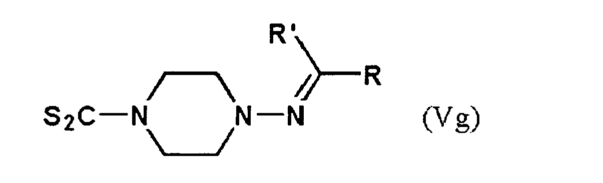 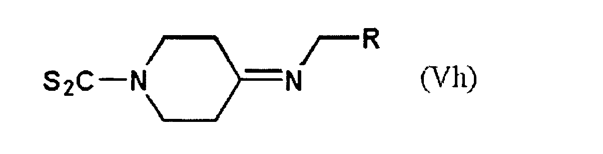 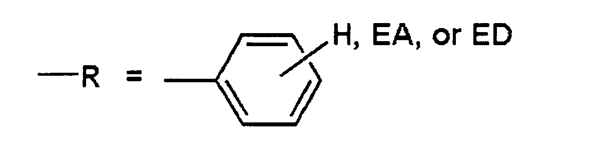 R' = H or CH₃
wherein H denotes an H-atom (i.e. the benzene ring is unsubstituted except where it joins the rest of the molecule), EA denotes an electron-accepting group, and ED denotes an electron-donating group. Preferably, said EA or ED group occupies the para- and/or meta-position with respect to the bond joining the benzene ring to the rest of the molecule. More preferably, said EA or ED group occupies the para-position.

In one embodiment said dithiocarbamate compound is selected from the group comprising the piperazine (X = N) or piperidine (X = CH) derivatives having the general structures: 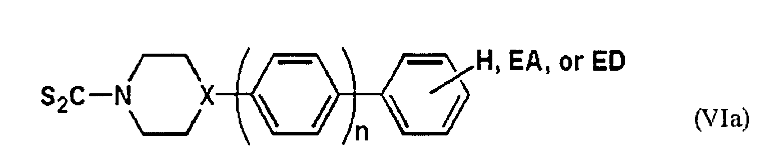 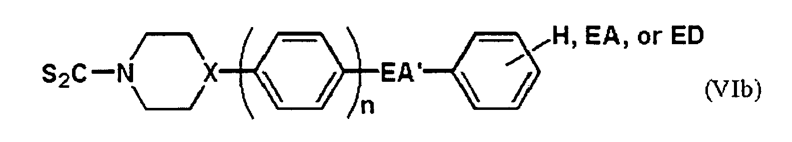 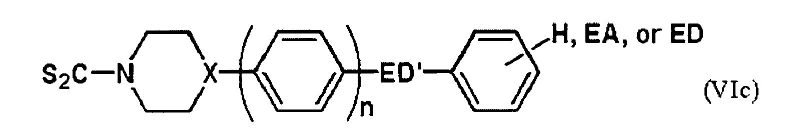 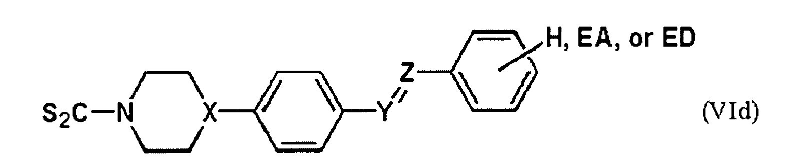 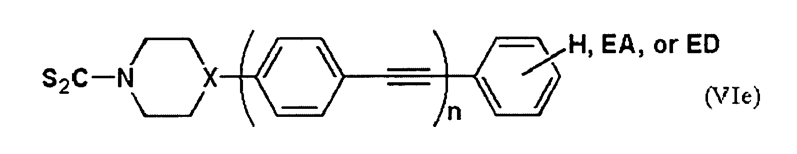 n=1-3
X=NorCH
Y = N, CH, or C(CH₃)
Z = N, CH, or C(CH₃)
wherein EA' denotes a bridging electron-accepting group selected from the group comprising -OC(O)-, -C(O)O-, -C(O)-, and -S(O)₂-, wherein ED' denotes a bridging electron-donating group selected from the group comprising -N(R')-, -O-, and -S-, and wherein H denotes an H-atom (i.e. the benzene ring is unsubstituted except where it joins the rest of the molecule), EA denotes an electron-accepting group, and ED denotes an electron-donating group. Preferably, said EA or ED group occupies the para- and/or meta-position with respect to the bond joining the benzene ring to the rest of the molecule. More preferably, said EA or ED group occupies the para-position.

In one embodiment said dithiocarbamate compound is selected from the group comprising benzylamine derivatives having the general structures: 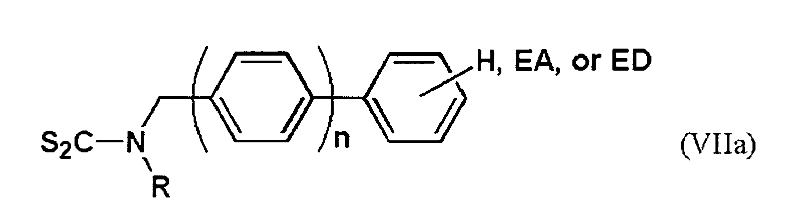 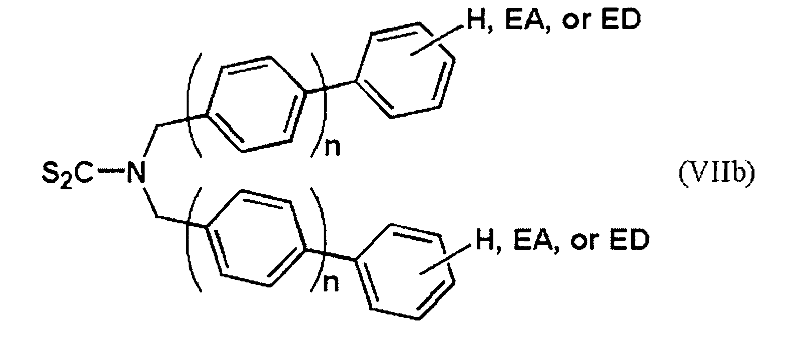 n=1-3
R = H, CH₃ or CH₂CH₃
wherein H denotes an H-atom (i.e. the benzene ring is unsubstituted except where it joins the rest of the molecule), EA denotes an electron-accepting group, and ED denotes an electron-donating group. Preferably, said EA or ED group occupies the para- and/or meta-position with respect to the bond joining the benzene ring to the rest of the molecule. More preferably, said EA or ED group occupies the para-position.

In one embodiment said dithiocarbamate compound is selected from the group comprising tetrahydroisoquinoline or isoindoline derivatives having the general structures: 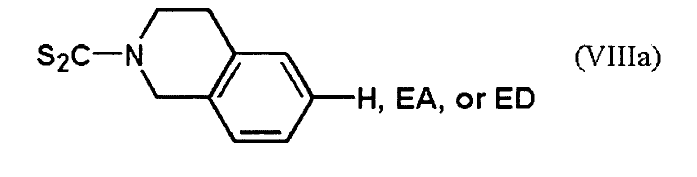 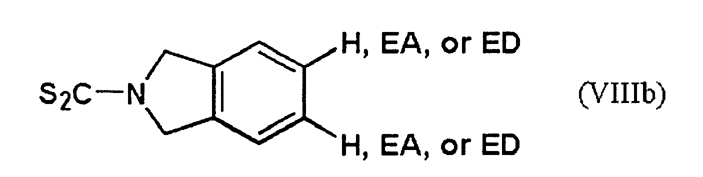 wherein H denotes an H-atom (i.e. the benzene ring is unsubstituted except where it joins the rest of the molecule), EA denotes an electron-accepting group, and ED denotes an electron-donating group.

In one embodiment said dithiocarbamate compound is selected from the group comprising aniline or diphenylamine derivatives having the general structures: 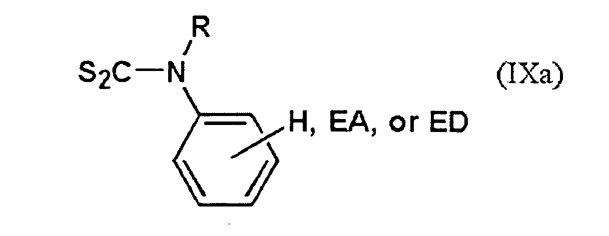 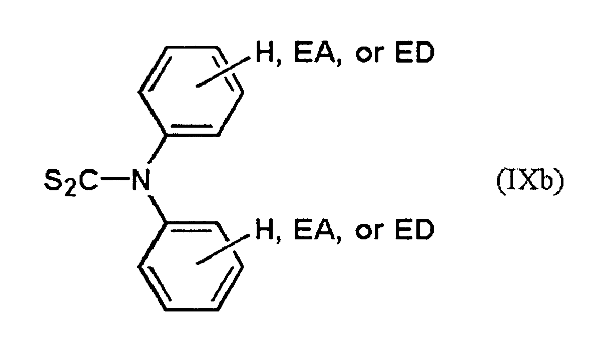 R = H, CH₃, CH₂CH₃, or CH₂C₆H₅
wherein H denotes an H-atom (i.e. the benzene ring is unsubstituted except where it joins the rest of the molecule), EA denotes an electron-accepting group, and ED denotes an electron-donating group. Preferably, said EA or ED group occupies the para- and/or meta-position with respect to the bond joining the benzene ring to the rest of the molecule.

In one embodiment said EA is a heteroaromatic group selected from the group comprising pyridine, pyrimidine, pyrazine, or triazine substituents, or boron trifluoride (BF₃) complexes thereof, having the general structures:

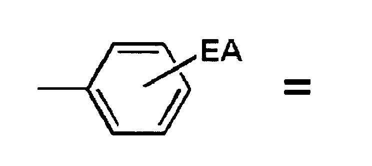

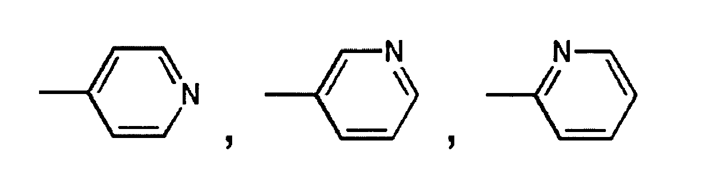

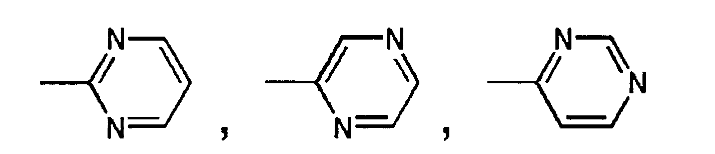

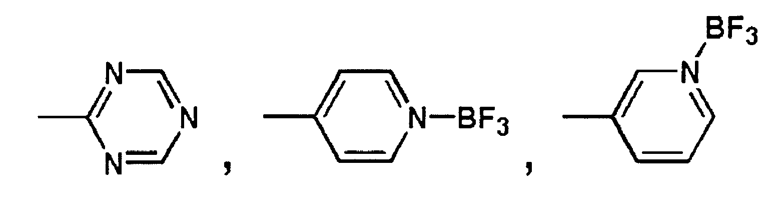

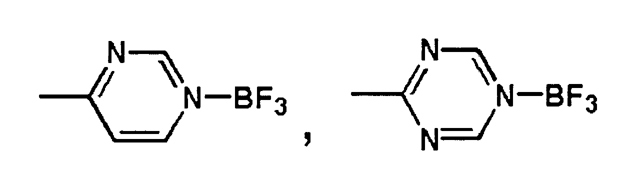

As is known from the prior art, the pi-conjugated systems in compounds such as those described above may be elongated to provide greater dipole moments, but the synthesis of such elongated molecules may be more demanding.

The substitution of an atom in a molecule with another atom or group of atoms, e.g. EA or ED, can affect both the direction and magnitude of the dipole moment of the molecule. This influence can be explained by two effects: the inductive effect and the conjugative (= resonance = mesomeric) effect. These effects are permanent effects that are present in the ground state of the molecule. An inductive effect involves the polarization of chemical bonds and involves only electrons in sigma bonds, while a conjugative effect is a result of p-orbital overlap (delocalization) and involves only electrons in pi bonds. Most elements other than metals and carbon have a significantly greater electronegativity than hydrogen. Consequently, substituents in which nitrogen, oxygen and halogen atoms form sigma-bonds to an aromatic ring exert an inductive electron withdrawal. The conjugative effect facilitates electron pair donation or withdrawal, to or from an aromatic ring, in a manner different from the inductive shift. If the atom bonded to an aromatic ring has one or more non-bonding valence shell electron pairs, as do nitrogen, oxygen and the halogens, electrons may flow into the ring by the conjugative effect. On the other hand, polar double and triple bonds conjugated with an aromatic ring may withdraw electrons.

The inductive and conjugative effects of particular EA or ED are related to the Hammett substituent constants (e.g. σ_{P} and σ_{M}) of the groups (Chem. Rev. 91, 165 (1991)). Electron-accepting (EA) groups, such as halogen atoms (F, Cl, Br, I) or groups containing halogen atoms, such as CF₃, COOCF₃, SO₂CF₃, or COCF₃, or groups containing double or triple bonds, such as CHO, COOCH₃, SO₂CH₃ SO₂NH₂, COCH₃, CN, or NO₂ generally have positive Hammett substituent constants. Likewise, replacement of one or more of the CH groups in a benzene ring by other groups or atoms such as nitrogen (N) to form heterocycles such as in pyridine (1 N atom), pyrimidine or pyrazine (2 N atoms), or triazine (3 N atoms), tends to make the pi-conjugated system a stronger electron acceptor. Electron-donating (ED) groups, such as NH₂, NHCH₃, N(CH₃)₂, OH, or OCH₃, generally have negative Hammett substituent constants.

Another concept for quantifying the electron-accepting or electron-donating properties of atoms or group of atoms is based on the electronegativities of elements. The electronegativity of an atom is a measure of the ability of the atom to attract electrons. Although it was originally defined by Pauling as an invariant property of an atom, it was later found that the electronegativity of an atom depends upon the chemical environment of the atom in a molecule, such as the hybridization of the atom and its oxidation state. Various methods have been proposed to estimate the "group electronegativity" of a group of atoms, such as a substituent replacing an H-atom on a benzene ring. Generally, the electronegativity of a group can be defined as a weighted average of the electronegativities of its constituent atoms, and electron-accepting (EA) groups have larger electronegativities than electron-donating (ED) groups (J. Phys. Chem. 70, 2086 (1966)).

It is advantageous to combine one or more electron-accepting (EA) groups and one or more electron-donating (ED) groups within the same molecule to obtain a compound with a net dipole moment (µ) larger than can be obtained with either type of group alone. For this purpose, EA and/or ED groups that serve to connect (via chemical bonding) parts of the molecule are required, which are referred to in the formulations above as "bridging groups". Suitable bridging groups with electron-accepting character (EA' in the above formulations) include the ester (-OC(O)- and -C(O)O-), carbonyl (-C(O)-), amide (-N(R')C(O)-), and sulfonamide (-S(O)₂N(R')- and -N(R')S(O)₂-) groups, while suitable bridging groups with electron-donating character (ED' in the above formulations) include the amine (-N(R')-), ether (-O-), thioether (-S-), and amide (-C(O)N(R')-) groups.

It should be noted that the classification of a particular substituent as electron-accepting or electron-donating may depend on its chemical environment. Thus, for example, the ester group -C(O)O- in the segment -C(O)O-C₆H₄-X is relatively electron-accepting when -X is a good electron-donating substituent such as -N(CH₃)₂, while it is relatively electron-donating when -X is a good electron-accepting substituent such as -NO₂.

"Push-pull" compounds are ones in which electron-accepting (EA) and electron-donating (ED) groups interact via a pi-conjugated system such that a partial intermolecular charge transfer occurs from the donor group to the acceptor group through the conjugated path. Intramolecular charge transfer induces an asymmetric polarization of the ground state and can provide push-pull compounds with large ground-state dipole moments. Typical push-pull compounds are derivatives of benzene, biphenyl, styrene, stilbene, phenylazobenzene ("azobenzene"), diphenylacetylene, and bithiophene. A number of the dipolar dithiocarbamate compounds provided as examples in the present invention can be considered as push-pull type compounds, where the dithiocarbamate group is appended to the molecule.

While the generalizations considered above can help to guide the design and optimization of dipolar dithiocarbamate compounds and assemblies thereof for use in electronic devices, detailed theoretical calculations and, ultimately, experimental testing are required.

As is known in the prior art, intermolecular electrostatic interactions may prevent or impede dipolar molecules from forming self-assembled monolayers with the preferred orientation and/or organization according to the present invention, even though the molecules have rod-like geometry and are terminated with a functional group suitable for chemisorption to the substrate. Thus, for example, the inventors have observed that the dithiocarbamate derivative of 4-(4'-cyanophenyl)piperidine yields low-density (coverage <0.5 monolayer) assemblies on gold substrates even though its dipole moment is not particularly high.

One way to circumvent such difficulties associated with intermolecular electrostatic interactions is use molecular layers comprising a mixture of a polar dithiocarbamate compound with a less-polar one. Such mixed monolayers can be prepared either by a one-step process, in which the two compounds are simultaneously adsorbed to the substrate, or by a two-step process, in which the two compounds are adsorbed sequentially.

Another way to circumvent difficulties associated with intermolecular electrostatic interactions also involves a two-step process: i) depositing a self-assembled monolayer of a relatively non-polar dithiocarbamate compound and ii) chemically converting the molecules within said monolayer into a polar dithiocarbamate compound. Several different kinds of chemical reactions may be employed in step ii). These include acid-base reactions and condensation reactions. A Lewis acid (A) is a compound that can accept a pair of electrons from a Lewis base (B) that acts as an electron-pair donor to form the complex A-B. An example is the complex (= "adduct" = "addition compound") formed between boron trifluoride (BF₃) and pyridine. A condensation reaction is one in which two molecules (or substituents thereon) combine with the loss of a small molecule such as water. An example is the condensation of a carbonyl (C=O) group with a primary amine (NH₂) group to form an imine (C=N) bond and H₂O.

As used herein, when referring to substituent groups, the terms "electron-accepting", "electron-withdrawing", "electron-pulling", and "electronegative" are equivalent. Likewise, the terms "electron-donating", "electron-releasing", "electron-pushing", and "electropositive" are also equivalent.

In one embodiment, said dithiocarbamate compound is a zwitterionic dithiocarbamate compound.

In one embodiment, said zwitterionic dithiocarbamate compound is selected from the group comprising piperazine (X = N) or piperidine (X = CH) derivatives having the general structures: 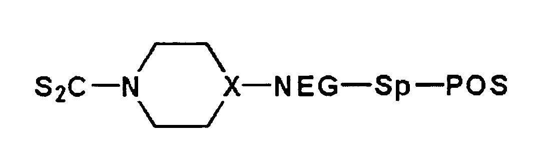 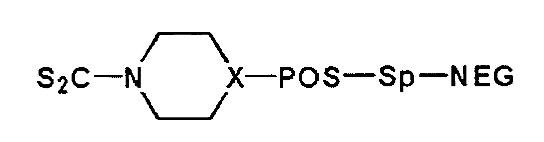 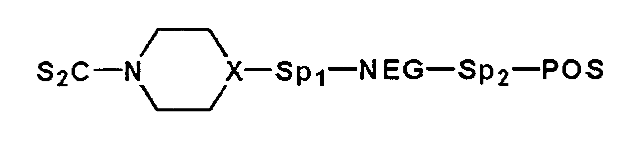 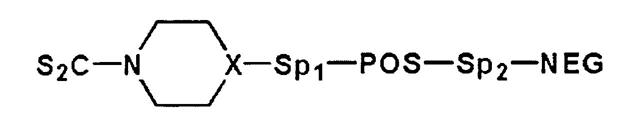 X = N or CH
wherein NEG denotes a negatively charged group, POS denotes a positively charged group, and Sp, Sp₁, and Sp₂ denote alkyl, aryl, or alkaryl connecting groups.

In another embodiment, said zwitterionic dithiocarbamate compound is selected from the group comprising piperazine (X = N) or piperidine (X = CH) derivatives having the general structures: 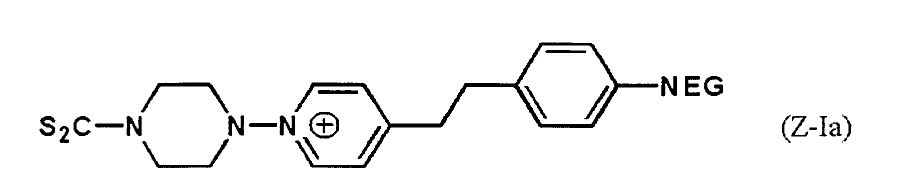 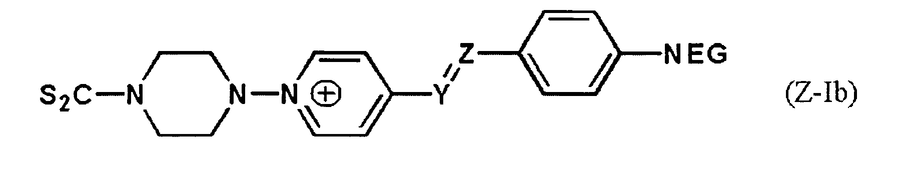 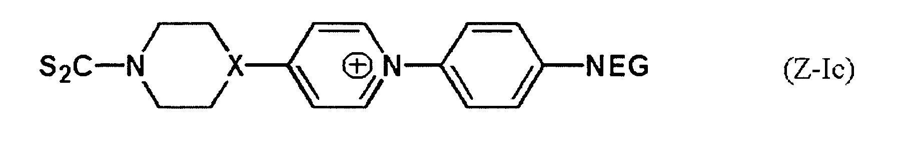 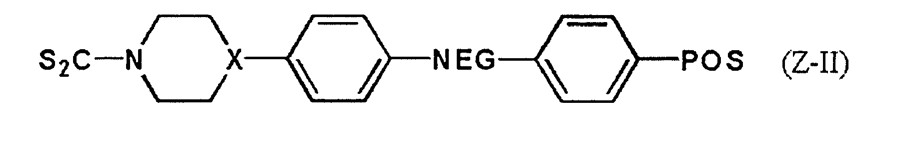 X = N or CH
Y = N, CH, or C(CH₃)
Z = N, CH, or C(CH₃)
wherein NEG denotes a negatively charged group and POS denotes a positively charged group.

A negatively charged group (NEG) is a group whose Lewis structure is formally negatively charged. In one embodiment, said NEG contains a formally negatively charged atom, preferably B, C, N, O, Si, or S. Examples of NEG are borate (-BO₃), trifluoroborate (-BF₃), carboxylate (-CO₂), dicyanomethide (-C(CN)₂), phenolate (-O), thiophenolate (-S), phosphonate (-PO₃), sulfonate (-SO₃), sulfonamidate (-SO₂N-), sulfonimidate (-NSO₂NH-), acylsulfonamidate (-CONSO₂-), tetrafluorosilicate (-SiF₄), imidazolate, pyrazolate, triazolate, and tetrazolate. In these formulae as shown, the negative charge has been omitted for reasons of simplicity.

A positively charged group (POS) is a group whose Lewis structure is formally positively charged. In one embodiment, said POS contains a formally positively charged atom, preferably N, O, P, or S. Examples are protonated primary, secondary or tertiary amino groups (e.g. NH₃, -N(R)H₂, or -N(R)₂H, wherein R denotes an alkyl, aryl, or alkaryl group) quaternary ammonium groups (including diazonium, imidazolium, piperidinium, pyridinium, pyrrolidinium, and thiazolium), quaternary phosphonium groups, tertiary oxonium (pyrilium) groups, and sulfonium groups.

Self-assembled monolayers (SAMs) of zwitterionic molecules wherein the negatively charged (anionic) group is the deprotonated form of an acid can be produced by a two-step process:
(i) First, the SAM is formed from the protonated, positively charged (cationic) form of the molecule (together with negatively charged counterion such as I⁻, ClO₄⁻, or PF₆⁻).
(ii) Second, the SAM is treated with a solution containing hydroxide ion (OH⁻), resulting in the SAM of the zwitterionic molecule plus H₂O.

Conversely, self-assembled monolayers (SAMs) of zwitterionic molecules wherein the positively charged (cationic) group is the protonated form of a base can be produced by a two-step process:
(i) First, the SAM is formed from the deprotonated, negatively charged (anionic) form of the molecule (together with positively charged counterion such as Li⁺, Na⁺, or N(CH₃)₄⁺).
(ii) Second, the SAM is treated with a solution of a mineral acid (such as HCl or H₂SO₄), resulting in the SAM of the zwitterionic molecule plus the mineral acid salt of the positively charged counterion.

The objects of the present invention are also solved by an assembly for use in an electronic device, said assembly comprising:
a) a conducting substrate, a semiconducting substrate, or an insulating inorganic substrate, said substrate having a surface,
b) a monolayer, preferably a self-assembled monolayer of at least one dithiocarbamate compound on said surface, wherein said monolayer, preferably said self-assembled monolayer is covalently bonded to said surface via an S₂CNH- or S₂CNR- group and is not in contact with another monolayer, preferably another self-assembled monolayer of at least one dithiocarbamate compound on the side opposite said surface, and wherein said dithiocarbamate compound is as defined above, and
c) an organic layer, an inorganic layer, or an electrolyte layer deposited on said monolayer, preferably on said self-assembled monolayer.

Various physical or chemical deposition methods for step c) are known in the art and include thermal evaporation, spin-coating, stamping, sputtering, atomic layer deposition, chemical vapor deposition, and electroplating.

The objects of the present invention are also solved by an electronic device comprising the assembly according to the present invention, wherein, preferably, said device is selected from a light-emitting device, a Schottky barrier diode, a rectifier, a field effect transistor, a photovoltaic device, a photochemical device, a memory device, a sensing device, or a display.

Another type of device in which dipolar dithiocarbamate compounds may be used to improve performance is the field emission display (FED). The primary component of the FED is the cold cathode. Various kinds of metals and semiconductors (molybdenum, silicon, hafnium, carbon, copper, zinc oxide, zinc sulfide, etc.) have been used as cold cathode emitters. Lowering the work function of the emitter by ∼2 eV can significantly lower the threshold electric field and allow a much higher current density (orders of magnitude) at a given electrical field. Chemisorption of a dithiocarbamate compound with a positive net dipole moment (µ) to the surface of an emitter will lower its work function. Stability of the dithiocarbamate layer is an issue in such an application (i.e., it must endure the elevated temperature required to effect a seal and reside passively within the vacuum environment without out-gassing or degrading during the entire operating life of the display). The dithiocarbamate group is intrinsically quite thermally stable. For example, thermogravimetric (TG) analyses of the sodium salts of phenylpiperazine and terphenylmethylamine dithiocarbamate derivatives showed that they were stable (under N₂) up to temperatures of 359 °C and 398 °C, respectively. Stability towards desorption and/or vaporization in vacuum from the surface of the emitter can be achieved by using molecules possessing more than one dithiocarbamate group, i.e. dithiocarbamate derivatives of oligomers or polymers or copolymers with primary or secondary amine groups in the repeating units, such as the compounds having the general structures P-I and P-II, where n > 1:

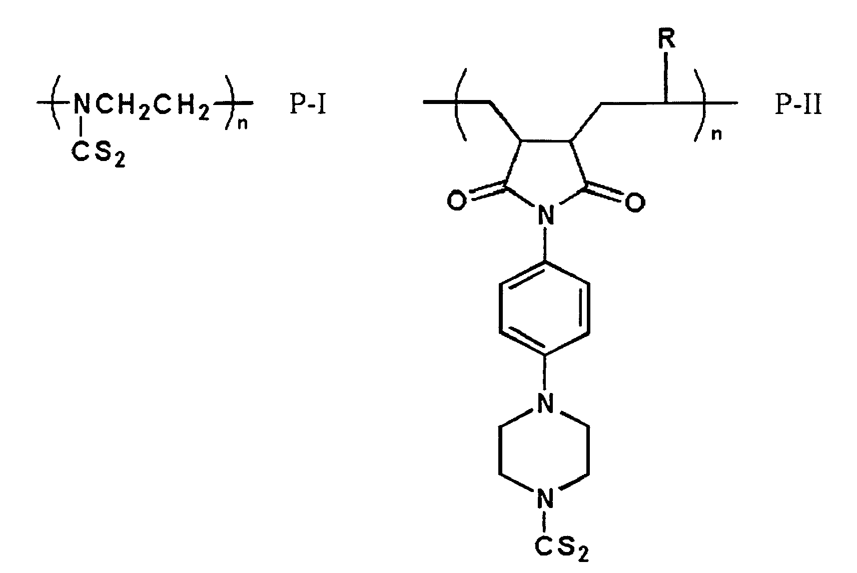

Compound P-I can be obtained by treating linear polyethyleneimine (PEI) with CS₂ and base. Compound P-II can be obtained by condensing an alternating copolymer of maleic anhydride with 1-(4-aminophenyl)piperazine to provide the imide, followed by treatment with CS₂ and base. The R-group in P-II depends on the copolymer constituent in the starting material and may be, for example, C₆H₅ (phenyl), COOCH₃, or OCH₃. The dipole moments of the dithiocarbamate-containing units of P-I and P-II, estimated by means of DFT calculations of model compounds, are +3.2 D and +4.9 D, respectively, so that chemisorption of these compounds to the surfaces of emitters comprising, for example, ZnO or Cu, should lower the work function of the emitters.

The objects of the present invention are also solved by a method of modifying the work function of a conducting, semiconducting, or insulating inorganic substrate, said method comprising the steps:
a) depositing a monolayer, preferably a self-assembled monolayer of said dithiocarbamate compound on a surface of said conducting, semiconducting, or insulating inorganic substrate, said dithiocarbamate compound, said substrate, said depositing step being as defined above, and
b) depositing an organic layer, an inorganic layer, or an electrolyte layer on said monolayer, preferably on said self-assembled monolayer.

As used herein, the term "substrate" is used is meant to refer to a solid, especially a solid inorganic substance, with a surface. Such a substrate may also be referred to herein as a "layer", especially when referring to stacked layered structures such as a metal/SAM/organic assembly, wherein all three materials comprise layers.

As used herein, the term "modifying" a work function of a substrate, is meant to refer to an increase or decrease in magnitude of said work function, or to relative shifts in the vacuum levels of two or more condensed phases, such as, for example, between the gate electrode and channel of a field-effect transistor.

As used herein, the term "work function" is meant to refer to the minimum work required for extracting an electron from the vacuum level of a conducting phase and placing it into vacuum just beyond the influence of electrostatic forces, i.e. into the so-called vacuum level. The term "dithiocarbamate compound", as used herein, in its broadest sense, is meant to refer to a compound having an S₂CNH- or S₂CNR- group in its molecular structure. The term "self-assembled monolayer" (or "SAM"), as used herein, is meant to refer to a two-dimensional film which comprises a single molecular layer and is covalently organized or assembled at a surface. Typically, this molecular assembly is formed by the adsorption of molecules on a solid surface. The adsorption can occur chemically (= chemisorption) or physically (= physisorption). As used herein, in the context of a self-assembled monolayer of dithiocarbamate compounds, such adsorption is preferably chemical. Chemisorption refers to the formation of a chemical bond between the adsorbate and the substrate involving a substantial rearrangement of electron density. The nature of this bond may lie anywhere between the extremes of vertically complete ionic or complete covalent character.

It is noted that the shorthand notation S₂CN<, where the symbol "<" represents connections of the N-atom to C-atoms of six-membered or five-membered rings, may be more appropriate than the shorthand notation S₂CNR- when referring to the dithiocarbamate groups of dithiocarbamate compounds of piperazine, piperidine, tetrahydroisoquinoline, or isoindoline derivatives.

The dithiocarbamate compounds in accordance with the present invention are preferably used in the form of salts, although other forms such as the acid or the disulfide can provide similar results. Typically such salts are prepared by reacting a primary or secondary organic amine with carbon disulfide (CS₂). Dithiocarbamate salts derived from secondary amines are generally more stable than those derived from primary amines and are therefore somewhat preferred. Examples of secondary amines from which such dithiocarbamate salts can be derived are piperazine and piperidine. A "dithiocarbamate derivative" of a particular amine is a dithiocarbamate compound which is prepared by reacting such particular organic amine with carbon disulfide. Preferred dithiocarbamate compounds in accordance with the present invention are the respective piperazine and piperidine dithiocarbamate compounds or dithiocarbamate derivatives. The term "uncharged polar component", as used herein, is meant to refer to a part or section of a molecule which, although electrically overall neutral, provides for a certain degree of polarity within such component. If a "second polarity" is "opposite a first polarity", this is meant to refer to the fact that the direction of the second polarity is opposite the direction of the first polarity. Likewise, if a "second polarity" is referred to as being "the same as a first polarity", this is meant to refer to the direction of both polarities which are therefore aligned in the same direction. This term is not meant to refer an identical magnitude of such two polarities.

As used herein, the terms "nonionic", "uncharged", "non-ionized", "no net charge", and "electrically neutral" are considered to be equivalent when referring to particular dithiocarbamate compounds or segments thereof. These considerations exclude the dithiocarbamate (S₂CNH- or S₂CNR-) group itself, which may be -1 charged or uncharged depending on the nature of bonding between the group and the substrate. Although the preferred nonionic compounds or segments thereof bear no net charge, they have distinct dipolar characteristics.

The present invention overcomes the shortcomings of the state of the art described above by using self-assembled monolayers (SAMs) of dithiocarbamate (DTC) compounds (preferably salts) to modify the work functions of electrodes. Dithiocarbamates are a versatile class of monoanionic 1,1-dithio ligands that are easily prepared from primary or secondary amines. Organic amines with a wide variety of polar substituents are known, many of which are commercially available, so that dithiocarbamate compounds are readily available to provide changes in work functions over a large range. The dithiocarbamate group forms coordination compounds (coordination complexes) with all d-block (transition) metals (e.g., Ti, Ni, Cu, Zn, Y, Zr, Pd, Ag, Cd, Hf, Pt, Au, Hg, etc.), s-block (alkali and alkaline earth) metals (e.g., Cs, Mg, Ca, Sr, etc.), f-block (lanthanide, actinide) metals (e.g., La, Ce, Nd, Eu, Tb, Er, U, etc.), as well as many of the p-block elements (e.g., B, Al, Si, P, Ga, Ge, As, Se, In, Sn, Sb, Pb, Bi, etc.). Since the atoms of these elements are present at the surfaces of many conductors, semiconductors, and insulators, dithiocarbamate compounds are able to chemisorb onto the surfaces of these materials by formation of a complex between at least one atom at the surface and the dithiocarbamate group ("surface chelation" or "surface coordination"). Due to charge delocalization within the dithiocarbamate group and its generally favorable electronic orbital overlap with orbitals of atoms of diverse elements, the barrier to charge transfer across the interface ("contact resistance") is relatively small compared to, e.g., thiols or oxy-acids. Furthermore, SAMs of dithiocarbamate compounds on gold, for example, are considerably more stable towards thermal desorption and oxidation then the corresponding thiol compounds.

Several experimental and theoretical studies were done internally by the inventors to provide a basis for the present invention. One objective of these studies was to compare self-assembled monolayers (SAMs) of dithiocarbamates with SAMs of thiols. Another objective was to prepare and characterize SAMs of dithiocarbamate derivatives of piperazine, piperidine, tetrahydroisoquinoline, isoindoline, benzylamine, and aniline.. These studies have mainly been done with gold substrates, but the results and conclusions are believed to be generally applicable to other substrates as well. Experimentally, measurements based on two photoelectron spectroscopic techniques, namely ultraviolet photoelectron spectroscopy (UPS) and x-ray photoelectron spectroscopy (XPS) (also known as electron spectroscopy for chemical analysis, ESCA), were made to obtain information about work function modification by SAMs (via UPS) and packing densities and compositions of molecules within SAMs (via XPS). XPS was also used to investigate the stabilities of the molecules towards thermal-induced desorption.

Examples of conducting substrates in accordance with the present invention are selected from metals such as Mg, Ca, Ni, Ag, Au, and Al and alloys thereof, and doped oxides such as tin-doped indium oxide (ITO) and fluorine-doped tin oxide (FTO); examples of semiconducting substrates in accordance with the present invention are selected from n-type (ZnO, TiO₂, SnO₂, WO₃) or p-type (NiO, Cu₂O) metal oxides, or the sulfides, selenides, arsenides, nitrides, or phosphides of d-block and p-block elements, such as GaAs; examples of insulating substrates in accordance with the present invention are selected from high dielectric constant (high-κ) materials (Al₂O₃, Y₂O₃, ZrO₂, La₂O₃, HfO₂, Ta₂O₅, SrTiO₃).

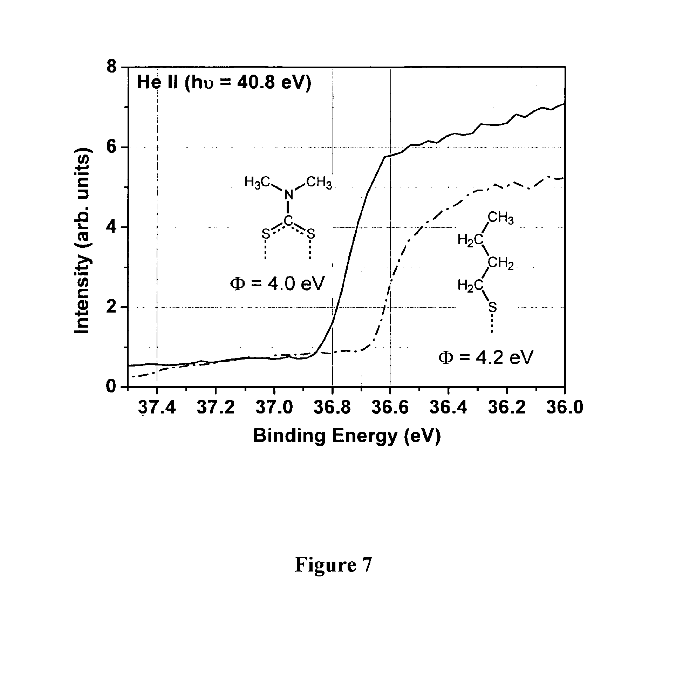

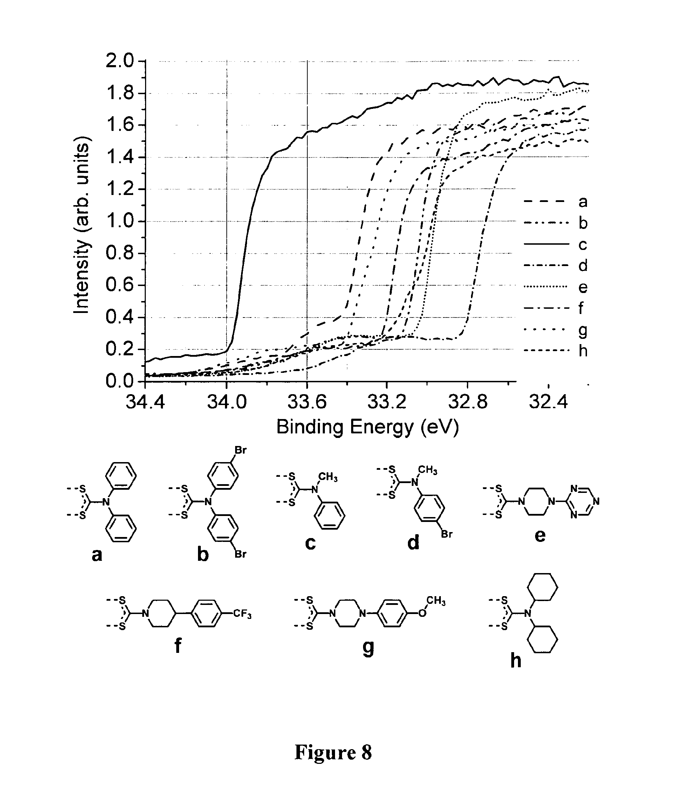

Various experiments were done to compare the effects of SAMs of dithiocarbamate and thiol compounds on the work function of gold. XPS measurements showed that both classes of compounds formed densely packed SAMs on gold surfaces. Ultraviolet photoelectron spectra of dimethyldithiocarbamate and *n*-butanethiol are shown in Figure 7, where the structures of the compounds are also indicated.. The spectra show that the ionization threshold energy (*E_{threshold}*) of the thiol is 0.2 eV lower than that of the dithiocarbamate. The work functions (Φ) for the two samples were obtained using the relationship Φ = *h*υ *-* (*E_{edge}* -*E_{threshold}.*), where *h*υ is the photoexcitation energy (40.8 eV) and *E_{edge}* is the energy of the Fermi edge. The value of *E_{edge}* is an instrumental parameter that is practically independent of the nature of the SAM. Referring to Figure 7, the values of Φ for the dithiocarbamate SAM, 4.0 eV, and for the thiol SAM, 4.2 eV, are approximately 1 eV lower than the work function of a clean Au(111) surface, 5.02 eV. The smaller change in work function caused by the thiol group compared to the dithiocarbamate group reflects the fact that the sulfur-gold bond in SAMs of thiols is nearly apolar (J. Phys. Chem. 110, 22628 (2006)). Figure 8 shows ultraviolet photoelectron spectra of eight dithiocarbamate compounds chemisorbed on gold, where, depending on molecular structure, the work function of the gold varies from 3.3 eV to 4.6 eV.

We obtained the dipole moments (µ) of the various dithiocarbamate compounds by means of density functional theory (DFT) calculations using the algorithm provided by the program Dmol³ (Accelrys, San Diego, CA). These calculations were performed within the non-local generalized gradient approximation using the BLYP functional with a double numerical basis set with polarization functions (DNP). Molecular bond lengths and electrostatic dipoles (in units of Debye) were obtained from the relaxed geometries. In order to simulate the conditions where the dithiocarbamate compounds are chemisorbed to gold (or silver) substrates, the molecular structures included an Au (or Ag) atom coordinated to the dithiocarbamate group. The calculated values are the gas phase dipole moments, i.e. for individual molecules in a vacuum environment. For a monolayer, a positive (+) dipole moment component µ leads to a decrease in the work function value. This is consistent with the trends in the work function values obtained from UPS data.

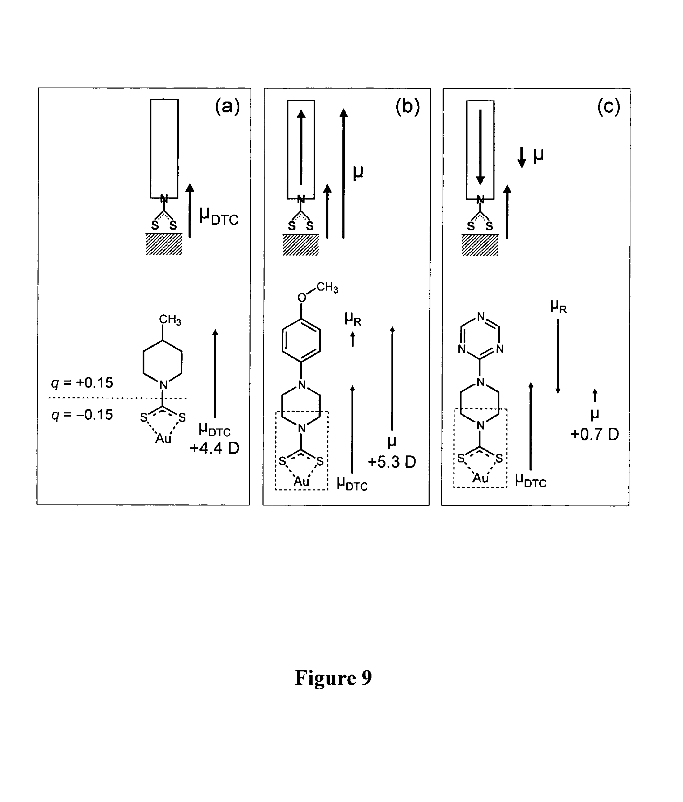

The results of several DFT calculations are shown in Figure 9. The molecular partial charge distribution (using Hirshfeld population analysis) of the dimethyldithiocarbamate molecule bound to a gold atom indicates that the net charges of the top and bottom halves of the system are positive and negative, respectively, with *q* = ±0.15 (Figure 9a). The dipole moment calculated based on this charge distribution, +4.4 D, is significantly larger than the value calculated for *n*-butanethiol, +1.8 D, where the symbol D represents the debye unit, which is defined according to the relationship 1 D = 0.208 *e*Å, *e* being the absolute charge of an electron. The results of calculations for two piperazine dithiocarbamate derivatives are also shown in Figure 9b and 9c. These two compounds are interesting because the para-methoxy substituent on the aromatic (phenyl) ring of the compound (Figure 9b) provides electron-donating character while the three nitrogen heteroatoms of the aromatic (triazinyl) ring of the compound (Figure 9c) provide electron-withdrawing character. The calculated dipole moment of the para-methoxy-phenyl compound is +5.3 D. If one assumes that the lower part of the molecule (plus gold atom) makes roughly the same contribution to the net µ as dimethyldithiocarbamate (µ_{DTC} ≅ +4.4 D), the contribution made by the remaining upper part of the molecule is µ_{R} ≅ +0.9 D. On the other hand, the calculated dipole moment of the triazinyl compound is only +0.7 D. In this case, the upper part of the molecule has a dipole moment µ_{R} ≅ -3.7 D pointing in the opposite direction to the lower part.

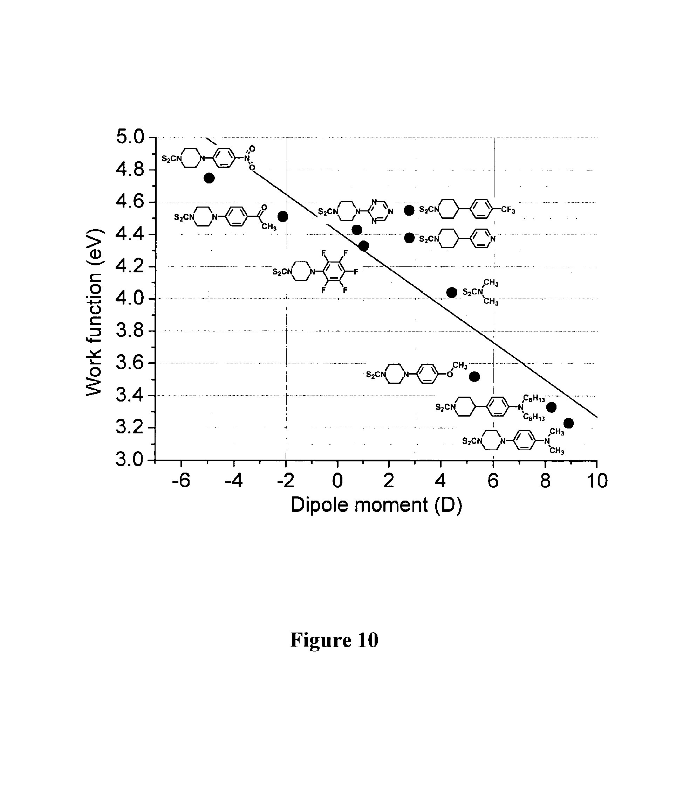

The plot in Figure 10 shows a roughly linear relationship between the dipole moment (µ) of ten dithiocarbamate compounds, calculated using DFT, and the work function (Φ) measured using UPS. For comparison, the upper and lower limits of work functions reported in the literature for SAMs of thiols on gold are 5.7 eV and 4.05 eV, respectively, and the work function of clean Au(111) is 5.0 eV. The work functions obtained with SAMs of the dihexylaminophenyl-piperidine and dimethylaminophenyl-piperazine dithiocarbamate compounds, 3.2-3.3 eV, lie between the work functions of Ca (2.87 eV) and Mg (3.66 eV), which are metals that are often used as cathodes in light-emitting diode devices but suffer from susceptability towards oxidation. Such difficulties may be avoided or lessened by using less cathodes of less reactive metals whose work functions have been decreased via chemisorption. The inventors are aware of only two examples in the prior art in which organic molecular layers were shown to decrease the work function of Au substrates to 3.3 eV. The electron-rich compounds used, 1,1-dimethyl-1H,1'H-[4,4']bipyridinylidene (Appl. Phys. Lett. 93, 243303 (2008)) and acridine orange base (Chem. Mater. (in press)), which lack functional groups for covalent chemisorption, were deposited by thermal evaporation in vacuum. The decrease in work function upon molecular adsorption of these compounds resulted from strong electric dipoles formed upon electron transfer from the molecules to the Au, which has been referred to as "molecular ionic chemisorption". The work function change could be achieved to some extent by varying the degree of surface coverage (i.e. sub-monolayer to monolayer). One advantage of using dithiocarbamate SAMs instead, as enabled by the present invention, is the opportunity to tune the work function via changes in chemical structure of the dithiocarbamate compound. As already noted, such changes can be made without much synthetic effort. A further advantage of the present invention is that the nature of the terminal organic residues attached to the amine nitrogen atoms of the piperidine or piperazine compounds can be changed without significantly affecting the work function of the metal. Thus, for example, the inclusion of a phenyl ring or substitution of methylene groups with oxygen atoms within said residues can be made to reduce the tunnelling barrier and/or surface energy of the resulting SAM to enable improved contact with the organic layer of the metal/SAM/organic assembly. Preferably, the static contact angle of water on the SAM (i.e., the inorganic/SAM surface) is within the range 30-140°; more preferably said contact angle is within the range 50-110°.

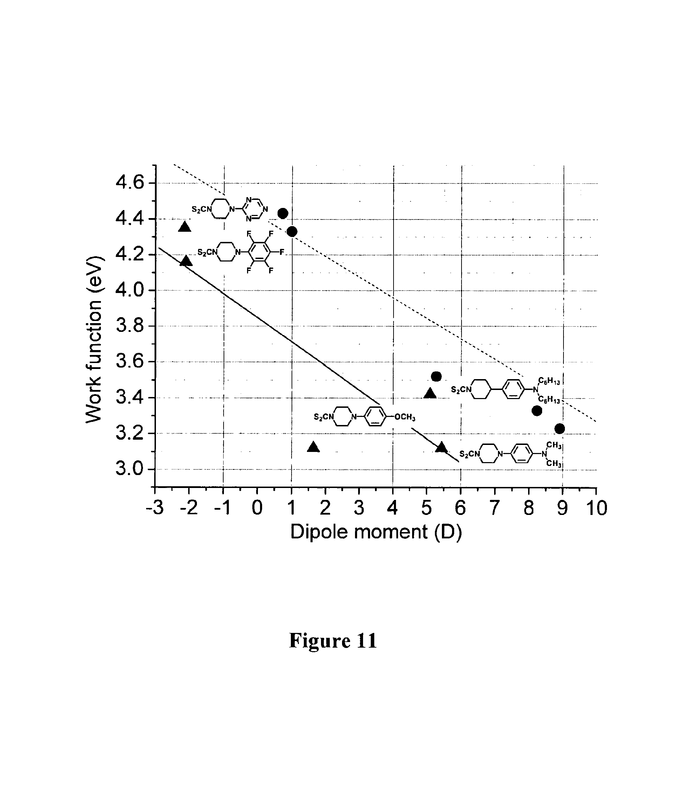

In addition to the above-described experiments with gold substrates, several experiments with silver substrates were performed to allow a direct comparison of monolayers of dithiocarbamate compounds on the two metals. The plot in Figure 11, like the one in Figure 10, shows the dependence of the work function (Φ), measured using UPS, on the dipole moment (µ), calculated using DFT, for five dithiocarbamate compounds on Ag (triangular data points) and Au (circular data points). Although the data for Ag are limited, it is apparent that the dipole moment of a given compound on Ag is approximately 3 D lower than the dipole moment on Au, resulting in a downward shift in the linear fit to the data; this trend may be related to the lower electronegativity of Ag compared to Au. XPS measurements of the same samples showed that the dithiocarbamate compounds formed densely packed monolayers on both metals. These results demonstrate that dithiocarbamate compounds with even modest (∼1 D) dipole moments can be used to lower the work function of silver from its intrinsic value (4.5 eV) to 3.1 eV, thereby providing cathodes for use in applications such as OLEDs.

The thermal stability of the metal/SAM interface is a key factor for the fabrication of molecular-based devices. The chemisorption energies of dimethyldithiocarbamate and *n-*butanethiol on Au(111) surfaces were determined by thermal desorption of their respective SAMs by monitoring the changes of the S(2p) and N(1s) XPS signals while the sample temperature was ramped from 300 K to 500 K in an UHV chamber. The desorption peaks of dimethyldithiocarbamate and *n*-butanethiol were located at temperatures of 450±10 K and 380±10 K, respectively. Based on the Redhead equation, these temperatures correspond to approximate desorption energies of 138±3 kJ/mol for dimethyldithiocarbamate and 119±3 kJ/mol for *n*-butanethiol. In the case of *n*-butanethiol, there was a shift in the S(2p) binding energy from 162 eV to 161 eV just before the onset of desorption (*T* ≈ 370-380 K), which could indicate the beginning of lateral diffusion of thiols on the gold surface. In contrast, the S(2p) signal of the dimethyldithiocarbamate SAM remained stable up to a temperature of ∼450 K. The higher chemisorption energy of the dithiocarbamate to gold is regarded as a consequence of its bidentate binding to the gold surface and is consistent with experiments showing that dithiocarbamate SAMs are stable towards displacement by thiols from solution (F. von Wrochem, Ph. D. Thesis, Universität Basel, 2007).

In accordance with preferred embodiments of the present invention, the dithiocarbamate compounds are used as dithiocarbamate salts. Dithiocarbamate salts are prepared by reacting primary or secondary organic amines with carbon disulfide (CS₂). The reaction is typically carried out in a polar solvent, such as ethanol or ethanol-water mixture, together with a base, such as sodium hydroxide (NaOH) or triethylamine. Dithiocarbamate salts derived from secondary amines are generally more stable than those derived from primary amines, and so are preferred. Secondary amines that are derivatives of piperazine or piperidine are particularly preferred for the present invention. Piperazine and piperidine compounds are characterized by six-membered rings containing either two nitrogen atoms or one, respectively, and their dithiocarbamate salts typically provide densely-packed self-assembled monolayers (SAMs) on electrode surfaces such as gold. Dense packing is advantageous because the change in work function of the surface upon adsorption of a SAM is directly proportional to the number of molecules per unit area in the monolayer if the orientation of the molecules does not change. The six-membered ring structure that is intrinsic to piperazine and piperidine compounds is advantageous because it provides a semi-rigid rodlike quality that helps maintain an upright molecular orientation in the SAM.

Deposition of monolayers, preferably of self-assembled monolayers (SAMs) of dithiocarbamate (DTC) compounds onto the surfaces of conducting, semiconducting, or insulating inorganic substrates is generally accomplished by exposing the substrates to solutions of the dithiocarbamate salts. The dithiocarbamate salts are generally synthesized by "in-situ" or "ex-situ" methods. The term "in-situ" refers to the fact that the dithiocarbamate salt is not synthesized and isolated prior to SAM deposition. Instead, the salt is formed directly in the solution used for SAM deposition by adding carbon disulfide to a solution of the organic amine precursor. As noted in the previous paragraph, a base such as sodium hydroxide or triethylamine is also added; otherwise the amine precursor itself serves as the base, but half of the precursor then becomes protonated and serves as the positively charged counterion for its negatively charged dithiocarbamate counterpart. The term "ex-situ" refers to the fact that solution used for SAM deposition is prepared using a previously synthesized and isolated dithiocarbamate salt. The concentration of dithiocarbamate salt in the solution for SAM deposition is typically in the range of 10⁻⁵ M to 10⁻² M, and the time allowed for SAM deposition to occur is typically in the range of 10⁻¹ h to 10² h. The main criteria in selecting the solvent for SAM deposition are the dithiocarbamate salt's solubility and stability in the solvent. Preferably, the solvent is neutral or basic since dithiocarbamate salts decompose under acidic conditions.

Alternatively, the deposition of self-assembled monolayers of dithiocarbamate compounds onto the surfaces conducting, semiconducting, or insulating inorganic substrates can be achieved by exposing said substrates to solutions of the compounds in their acid forms (characterized by the HS₂CN- group) or in their disulfide forms (characterized by the -NC(S)S-SC(S)N- group).

In accordance with the present invention, the dithiocarbamate compounds can be used to modulate the work functions of inorganic conductors, semiconductors, or insulators. Dithiocarbamates are easily prepared from primary or secondary amines having a wide variety of polar or, if desired, non-polar, substituents. Moreover, the contacts provided by a dithiocarbamate group, have relatively low electrical resistances as a result of charge delocalisation and generally favorable electronic orbital overlap during chemisorption. Self-assembled monolayers (SAMs) of dithiocarbamates have greater stability then SAMs of thiols, especially on noble metals, with respect to both thermal desorption and oxidation. The sulfur-metal bond in the dithiocarbamate anchoring group is strongly dipolar and contributes significantly to the net dipole moment in such dithiocarbamate SAMs. This feature makes it easier to achieve relatively large negative shifts in work functions with dithiocarbamates than with other anchoring groups. As shown by our results, dithiocarbamate derivatives of secondary amines, such as piperazine or piperidine, with a polar (µ > 4 D) rod-like segment attached to the dithiocarbamate group can form SAMs wherein the molecules are densely packed and have close to perpendicular (vertical) orientation with respect to the substrate (θ ≈ 0°), thereby providing reproducible, predictable and maximal shifts in work function. Preferably, the total coverage of the substrate by the molecular layer is within the range 0.7-1 monolayer. Preferably, the tilt angle θ is within the range 0-45 degrees (0-45%); more preferably θ < 30%.

The term "rod-like segment" as used herein is meant to refer to an elongation of a dithiocarbamate compound connected to the S₂CN- group through the N-atom and directed away from the S-atoms whose rod-like shape is achieved by connecting non-conjugated (σ) and/or pi-conjugated (π) groups in a linear manner and using groups with similar cross-sectional areas commensurate with the dithiocarbamate group itself. The preferred area per dithiocarbamate molecule on the substrate surface is within the range 0.2-0.7 nm²; more preferably said surface area per molecule is within the range 0.2-0.4 nm².

In one embodiment of the present invention, the structure of the dithiocarbamate compound has a plane of symmetry (also known as a plane of reflection or mirror plane), wherein said plane is perpendicular to the plane defined by the four atoms of the dithiocarbamate (S₂CN-) group and bisects the S-C-S angle of said group.

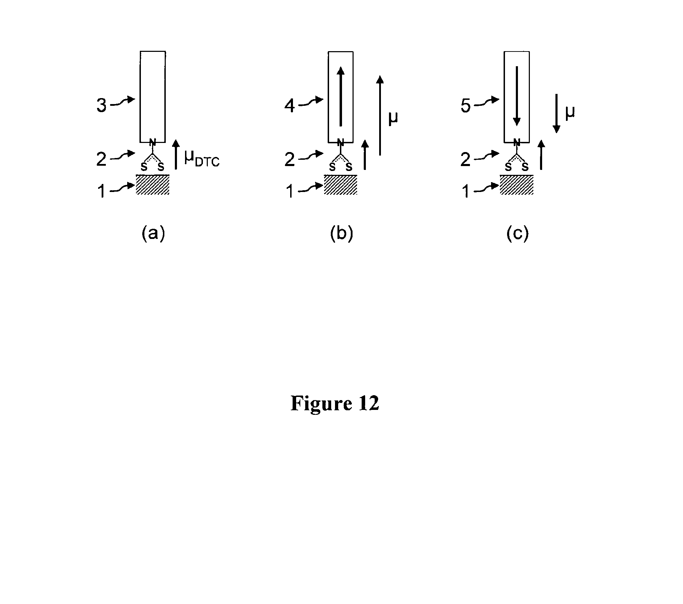

Figure 12 shows schematic representations of three preferred embodiments of the present invention, comprising an inorganic substrate, 1, which may be either electrically conducting, semiconducting, or non-conducting (insulating), and a chemisorbed and vertically oriented dithiocarbamate compound comprising a dithiocarbamate (S₂CNH- or S₂CNR-) group, 2, with an attached, preferably rod-like, segment. In example (a), the dithiocarbamate compound 3 has a relatively non-polar segment, so that the net dipole moment µ is mainly due to the dipole moment of the dithiocarbamate group µ_{DTC}. In example (b), the compound 4 has a polar segment whose dipole moment points in the same direction as µ_{DTC}, whereas in example (c) the compound 5 has a polar segment whose dipole moment points in the opposite direction as µ_{DTC}.

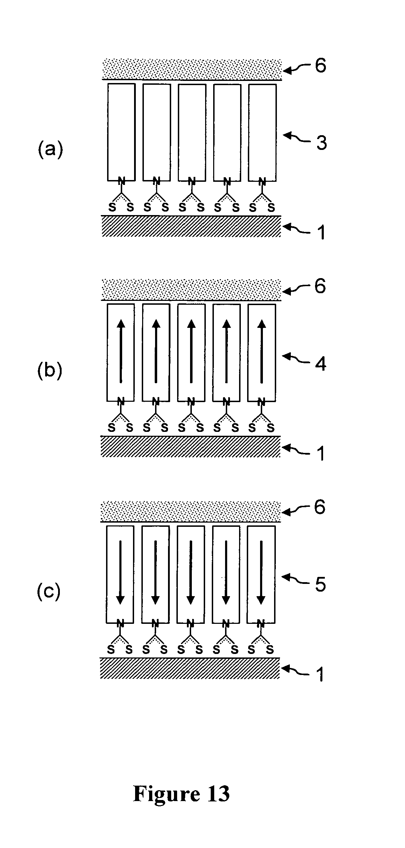

Figure 13 shows schematic representations of three preferred embodiments of the present invention, comprising an inorganic substrate 1, a SAM of a vertically oriented dithiocarbamate compound 3, 4, or 5 deposited on said substrate, and an organic layer 6 deposited on said SAM. The dipole moment of the SAM comprising 3 is mainly due to µ_{DTC} and points away from the substrate, thereby shifting its work function to a lower value. The dipole moment of the SAM comprising 4 points away from the substrate and thereby shifts its work function to a lower value, whereas the dipole moment of the SAM comprising 5 points towards the substrate and thereby shifts its work function to a higher value.

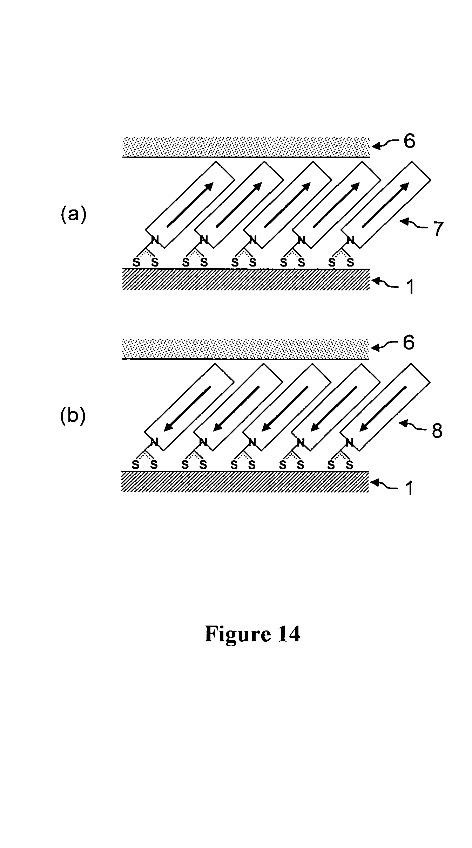

Figure 14 shows schematic representations of two preferred embodiments of the present invention, comprising an inorganic substrate 1, a SAM of a tilted dithiocarbamate compound 7 or 8 deposited on said substrate, and an organic layer 6 deposited on said SAM. The dipole moment of the SAM comprising 7 points away from the substrate and thereby shifts its work function to a lower value, whereas the dipole moment of the SAM comprising 8 points towards the substrate and thereby shifts its work function to a higher value. The tilt angle θ shown, 45°, results in a ca. 30% reduction in the dipole moment vector normal to the substrate compared to the SAM with vertically oriented molecules (θ = 0°).

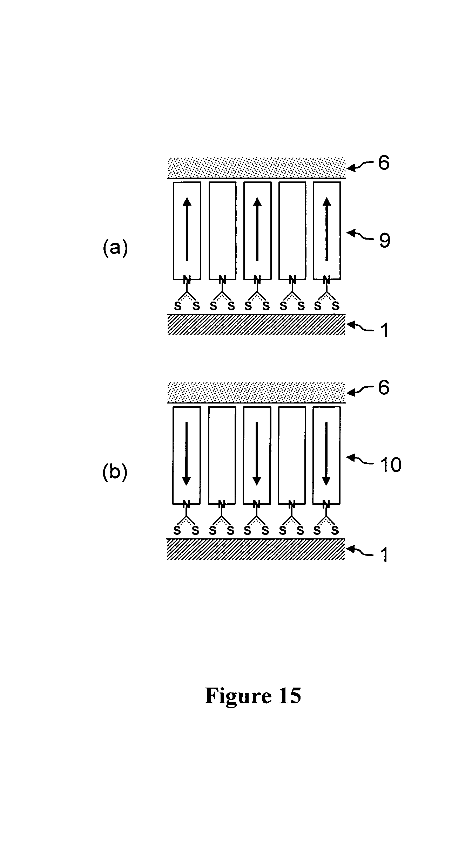

Figure 15 shows schematic representations of two preferred embodiments of the present invention, comprising an inorganic substrate 1, a SAM 9 or 10 comprising a mixture of vertically oriented dithiocarbamate compounds deposited on said substrate, and an organic layer 6 deposited on said SAM. One of the dithiocarbamate compounds in SAM 9 or 10 is polar and the other compound is relatively non-polar. Diluting the polar compound with the less polar one can attenuate electrostatic interactions between the polar groups and thus facilitate vertical orientation of all the molecules within the SAM.

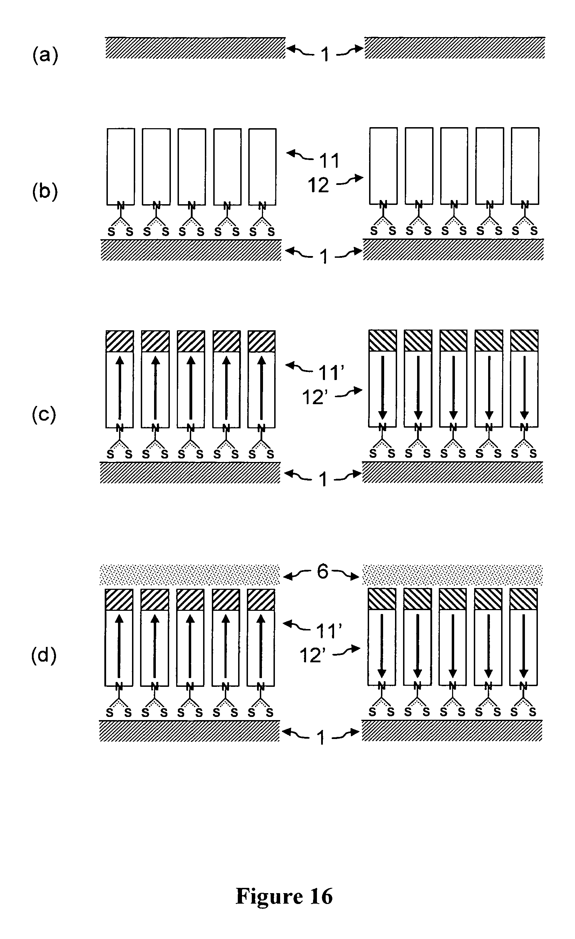

Figure 16 shows schematic representations of two preferred embodiments of the present invention, comprising (a) an inorganic substrate 1, (b) a SAM of vertically oriented precursor dithiocarbamate compound 11 or 12 deposited on said substrate, (c) a SAM of said precursor compound following chemical conversion to a more polar dithiocarbamate compound 11' or 12', and (d) an organic layer 6 deposited on said SAM following said chemical conversion.

The following Example is an implementation of steps (a)-(c) of Figure 16. To an ethanolic solution of 1-pyridin-4-yl-piperidine (0.010 M, 0.20 mL) were added successively ethanolic solutions of carbon disulfide (0.10 M, 0.020 mL) and sodium hydroxide (0.10 M, 0.020 mL), to form "in-situ" the dithiocarbamate derivative of the piperazine compound (4-(pyridin-4-yl)piperidine-1-carbodithioate). A template-stripped gold substrate (1) was dipped into this solution and kept there for 23 hours to provide a SAM of the dithiocarbamate derivative (11). XPS analysis of the SAM indicated a molecular density that was 0.63 that of alkanethiols, indicating that the molecules were approximately vertically oriented. The calculated dipole moment of the compound, attached to a gold atom via the dithiocarbamate group, was +2.76 D and the work function of the sample determined by UPS was 4.38 eV. The sample was dipped into a solution of boron trifluoride etherate (0.10 M in diethyl ether) for 15 minutes to form the BF₃ complex of the molecule in the SAM (11'). The calculated dipole moment of the compound having the BF₃ group complexed to the pyridine nitrogen atom, attached to a gold atom via the dithiocarbamate group, was -7.78 D, i.e. larger in magnitude and opposite in sign to the dipole moment of the compound without BF₃, and the work function of the sample determined by UPS was 5.07 eV. This example thus illustrates the concept of forming a SAM of a precursor compound (i.e., 4-(pyridin-4-yl)piperidine-1-carbodithioate) and chemically converting it into a more polar SAM (i.e., the boron trifluoride complex of 4-(pyridin-4-yl)piperidine-1-carbodithioate).

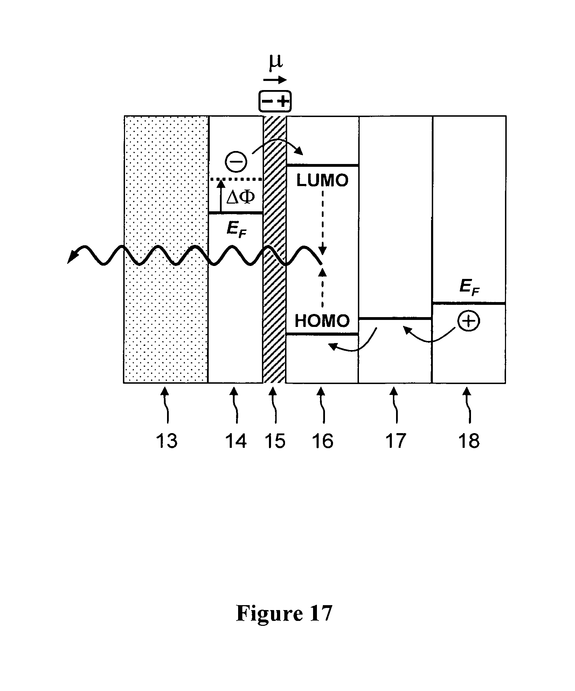

Figure 17 shows a schematic diagram of a preferred embodiment of the present invention representing an organic light-emitting diode (OLED) comprising six layers: 13, an optically transparent support, preferably glass or polymer;
14, an optically transparent or semitransparent cathode, preferably gold or silver (20-30 nm thick) with an intermediate layer of titanium or chromium (2-3 nm thick) for improved adhesion;
15, a monolayer of a dipolar dithiocarbamate compound whose dithiocarbamate group is chemisorbed to 14 and whose electrical dipole is directed away from the surface of 14, resulting in a decrease in the work function;
16, an electron conducting light emitter;
17, a hole conductor; and
18, an anode.
Dithiocarbamate monolayers (15) can reduce the work function of clean Au or Ag (14) (whose Fermi level *E_{F}* is indicated by the continuous line) to values as low as 3.0 eV (indicated by the dotted line). In this embodiment, the Fermi level of the modified electrodes is aligned with the LUMO of the electron conducting light emitting layer (16), thereby reducing the electron injection barrier from the cathode to the electron conducting organic layer considerably and, as a consequence, reducing the operating voltage of the OLED and increasing its luminance efficiency.

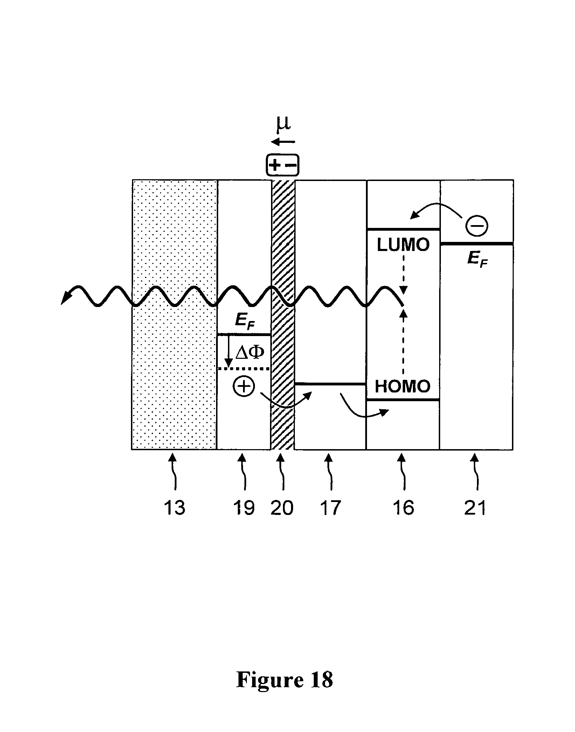

Figure 18 shows a schematic diagram of a preferred embodiment of the present invention representing an organic light-emitting diode (OLED) comprising six layers:
13, an optically transparent support, preferably glass or polymer;
19, an optically transparent or semitransparent anode, preferably fluorine-doped tin dioxide (FTO), or tin-doped indium oxide (ITO), or gold or silver (20-30 nm thick) with an intermediate layer of titanium or chromium (2-3 nm thick) for improved adhesion;
20, a monolayer of a dipolar dithiocarbamate compound whose dithiocarbamate group is chemisorbed to 19 and whose electrical dipole is directed towards the surface of 19, resulting in an increase in the work function;
17, a hole conductor;
16, an electron conducting light emitter; and
21, a cathode, preferably aluminum with an intermediate layer of lithium fluoride (0.1-1 nm thick), or calcium or magnesium.

In this embodiment, the dithiocarbamate monolayer 20 increases the work function of the anode (19) (whose Fermi level *E_{F}* is indicated by the continuous line) to a value in the range 5.0-5.5 eV (indicated by the dotted line). The Fermi level of the modified electrodes is thereby aligned with the HOMO of the hole conducting organic layer (17), reducing the electron injection barrier and consequently reducing the operating voltage of the OLED and increasing its luminance efficiency.

The choice of various end groups on the dithiocarbamate compound in the monolayer (15 or 20) allows the surface energy of the modified electrode (14 or 19) to be tuned so as to improve the adhesion properties of the organic layer (16 or 19) with the modified electrode. Good adhesion can improve device stability by reducing the probability of pinholes and delamination. Similar advantages of electrode modification with monolayer 15 or 20 apply if inorganic or organic-inorganic composite materials are used instead of organic materials for the electron conducting and/or hole conducting layers (16 and/or 17).

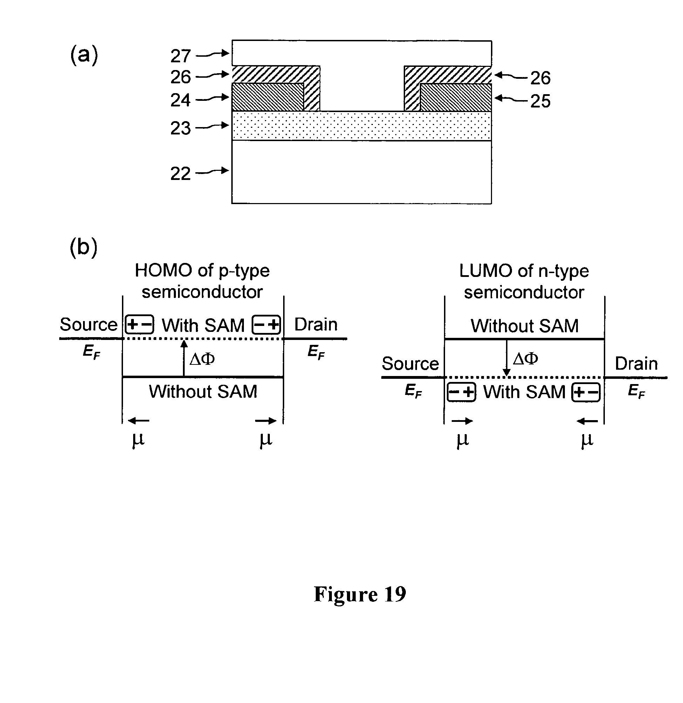

Figure 19 (a) shows a schematic diagram of a preferred embodiment of the present invention representing a field-effect transistor (FET) or TFT (thin film transistor) comprising six layers: 22, a gate electrode;
23, an insulator;
24, a source electrode, preferably gold or silver;
25, a drain electrode, preferably gold or silver;
26, a monolayer of a dipolar dithiocarbamate compound whose dithiocarbamate group is chemisorbed to 24 and 25; and
27, a semiconductor.

The diagrams in Figure 19 (b) illustrate the preferred embodiments depending on whether the semiconductor 27 in the channel is p-type or n-type. In the case of a p-type semiconductor, a dithiocarbamate monolayer (26) is used whose electrical dipole is directed towards the surface of the source electrode (24) and the drain electrode (25), resulting in an increase in the work function and bringing the HOMO level of the semiconductor in line with the Fermi energy level of the electrode (indicated by the dotted line). In the case of an n-type semiconductor, a dithiocarbamate monolayer (26) is used is used whose electrical dipole is directed away from the surface of the source electrode (24) and the drain electrode (25), resulting in a decrease in the work function and bringing the LUMO level of the semiconductor in line with the Fermi energy level of the electrode (indicated by the dotted line). In both cases, the charge injection barrier between the source or drain electrodes and the organic semiconductor is reduced, decreasing the threshold voltage of the transistor and increasing the source-drain current at given gate voltage.

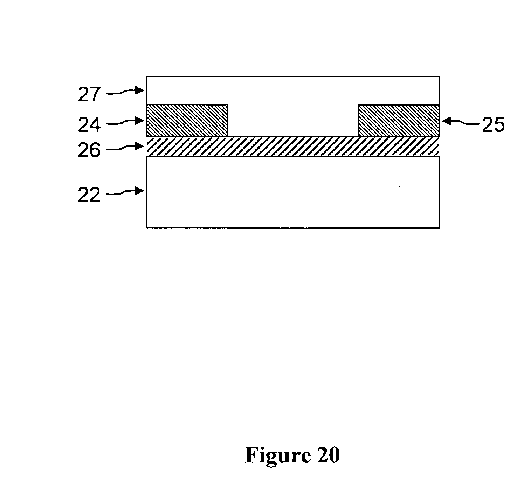

Figure 20 shows a schematic diagram of a preferred embodiment of the present invention representing a field-effect transistor (FET) or TFT (thin film transistor) comprising five layers:
22, a gate electrode;
26, a monolayer of a dipolar dithiocarbamate compound whose dithiocarbamate group is chemisorbed to 22;
24, a source electrode;
25, a drain electrode; and
27, a semiconductor.

In this embodiment, the dithiocarbamate monolayer (26) is used as a thin insulating layer (0.5-3 nm) between the gate electrode (22) and both the organic semiconductor (channel) (27) and the source/drain electrodes (24/25) of the device. The molecular backbone of the dithiocarbamate compound in 26 consists of structures having a high electronic bandgap, this way blocking the charge transport from drain/source electrode to the gate electrode. The very thin organic insulating layer implies a high electrical field in the channel region of the organic semiconductor when a bias is applied to the gate electrode, thus allowing the device to be operated at low source-drain voltages. In this embodiment, the vacuum level shift between the gate electrode and the organic semiconductor induced by the monolayer can be used to increase the field-effect mobility of the charge carriers in the semiconductor channel. The high stability of the dithiocarbamate monolayer is a major factor enhancing the robustness (towards defects and temperature) of the gate insulator.

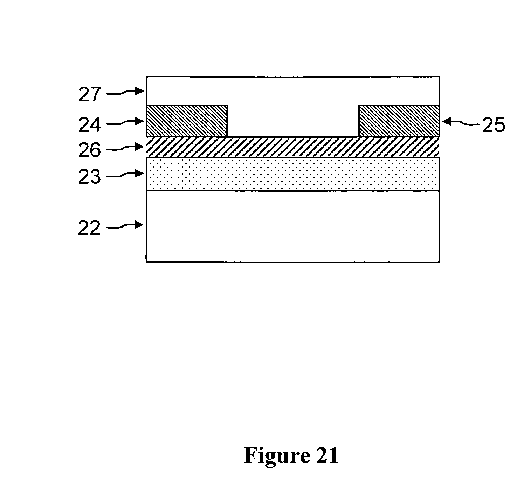

Figure 21 shows a schematic diagram of a preferred embodiment of the present invention representing a field-effect transistor (FET) or TFT (thin film transistor) comprising six layers:
22, a gate electrode;
23, an insulator, preferably a high-k transition metal oxide;
26, a monolayer of a dipolar dithiocarbamate compound whose dithiocarbamate group is chemisorbed to 23;
24, a source electrode;
25, a drain electrode; and
27, a semiconductor.

In this embodiment, the dithiocarbamate monolayer (26) is used as a dipolar layer on top the gate insulator, i.e. it is applied between the gate insulator 23 and both the organic semiconductor (channel) (27) and the source/drain electrodes (24/25) of the device. The vacuum level shift between the gate electrode and the organic semiconductor induced by the monolayer is used to increase the field-effect mobility of the charge carriers in the semiconductor channel.

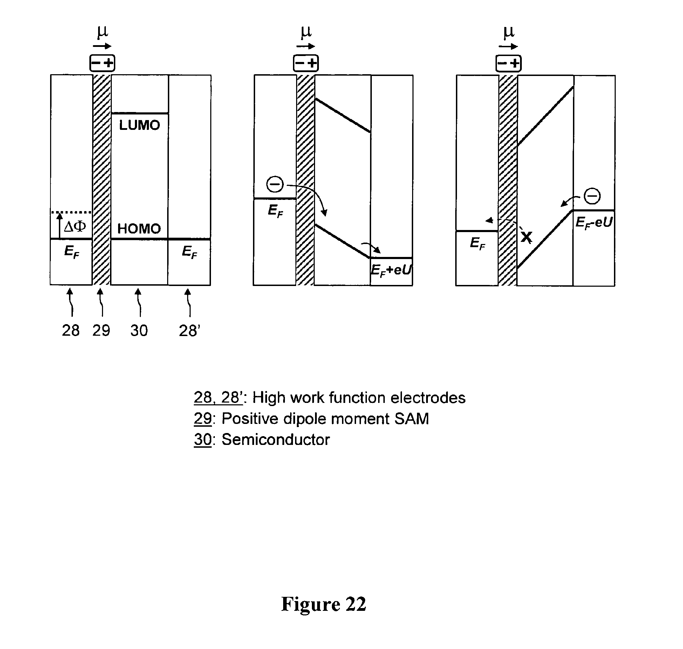

Figure 22 shows schematic diagram of a preferred embodiment of the present invention representing a diode or rectifier comprising four layers:
28, 28', left and right (or top and bottom) electrodes made of the same conductive material;
29, a monolayer of a dithiocarbamate compound with a positive dipole moment whose dithiocarbamate group is chemisorbed to electrode 28; and
30, a semiconductor.

The diagram on the left hand side of Figure 22 shows the device at zero applied potential. The SAM (29) comprises a dipolar dithiocarbamate compound whose net dipole moment is directed away from the electrode, thereby shifting the electrode's Fermi energy upwards to a position indicated by the dotted line. The diagram in the middle of the figure shows the device with a positive potential applied to the right electrode, whereby electron transport can freely occur from left to right (hole transport can freely occur from right to left). The diagram on the right side of the figure shows the device with a negative potential applied to the right electrode. In this case, an energy barrier to electron transport exists. Thus the asymmetry in charge injection barriers with respect to the two electrodes due to the presence of the SAM on one of them results in rectification of the current through the device.

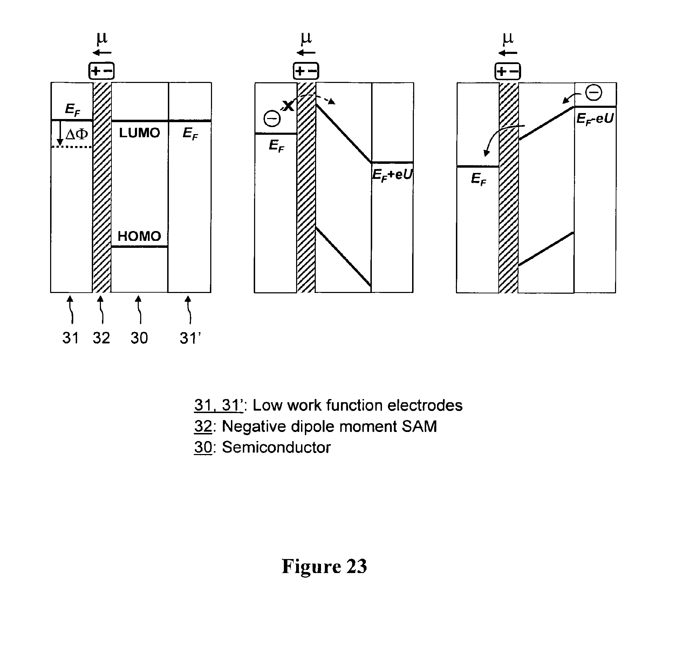

Figure 23 shows a schematic diagram of a device similar to that in Figure 22 except that the dithiocarbamate compound comprising the SAM (32) has a negative dipole moment, i.e., the net dipole moment of the is directed towards the left electrode. The diagram on the left hand side of the figure shows the device at zero applied potential. The Fermi energy level of the left electrode (31) is shifted downwards due to the SAM to a position indicated by the dotted line.
The diagram in the middle of the figure shows the device with a positive potential applied to the right electrode. In this case, an energy barrier to electron transport from the left electrode to the LUMO level of the semiconductor (30) exists. The diagram on the right side of the figure shows the device with a negative potential applied to the right electrode, whereby electron transport can freely occur from right to left (hole transport can freely occur from left to right). Thus the asymmetry in charge injection barriers with respect to the two electrodes due to the presence of the SAM on one of them results in rectification of the current through the device, in a direction opposite to that in the device in Figure 22.

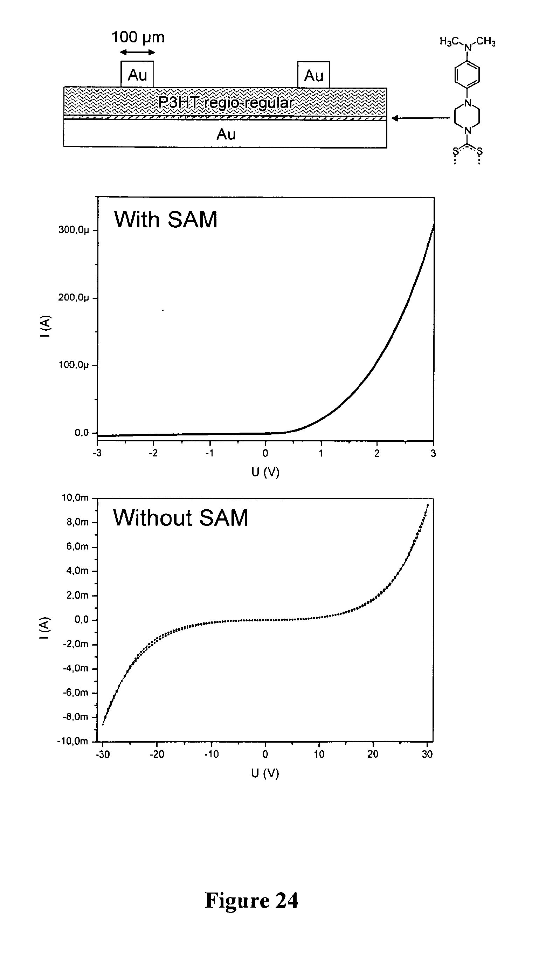

Figure 24 shows an Example of implementation of the embodiment in Figure 22. A crossbar array with gold electrodes was used for the device in this example, whose design is indicated in the diagram at the top of the figure. A monolayer of a dithiocarbamate derivative whose structure is also indicated was deposited by self-assembly onto the bottom electrodes. The calculated dipole moment of this compound (attached to a gold atom) was +8.91 D; the work function of gold with a SAM of this compound determined by UPS was 3.23 eV. The semiconductor used in the device was regio-regular poly(3-hexylthiophene) (P3HT). The gold top electrodes were deposited by evaporation. The diagram in the middle of Figure 24 clearly shows rectification in the current-voltage behavior of the device with the SAM (with the bottom electrodes being grounded). The rectification ratio and current density at 3 V were 100:1 and 6 A/cm², respectively. The diagram at the bottom of Figure 24 shows the symmetric current-voltage behavior of a control device prepared without the SAM.

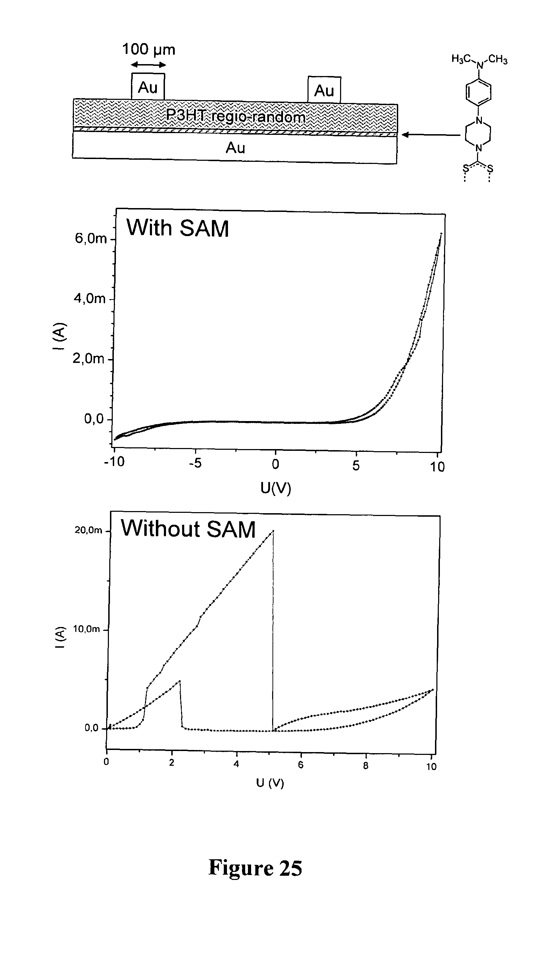

Figure 25 shows another Example of implementation of the embodiment in Figure 22. The device structure is the same as that of the previous Example except that the semiconductor used was regio-random poly(3-hexylthiophene) (P3HT). The diagram in the middle of Figure 25 clearly shows rectification in the current-voltage behavior of the device with the SAM (with the bottom electrodes being grounded). The rectification ratio and current density at 3 V were 10:1 and 0.2 A/cm², respectively. The diagram at the bottom of Figure 25 shows the irregular current-voltage behavior of a control device prepared without the SAM, indicating the formation and breaking of metal filaments. Such behavior was not observed in the device with the SAM even at applied voltages up to 50 V.

The following Examples as shown by tables 1-15 summarize the calculated values of the net dipole moment (µ) and its the x-axis vector (µₓ) for various non-ionic and zwitterionic dithiocarbamate compounds according to the present invention. The values of µ and µₓ were obtained using density-functional theory (DFT) calculations. A gold atom is attached to the sulfur atoms of the dithiocarbamate group for calculation purposes to simulate a gold surface and is not part of the dithiocarbamate compounds in accordance with the present invention. The net moment µ is determined from its x-, y-, and z-vector components according to µ = (µₓ² + µ_{y}² + µ_{z}²)^{1/2}, wherein the x-vector component is directed approximately along the long axis of the molecule and is thus the component most responsible for shifting the work function of the substrate. These Examples demonstrate that a wide range of dipole moments are possible with dithiocarbamate compounds, meaning that the dipole moments, and consequently shifts in work function of substrates to which the compounds are chemisorbed, can be finely tuned. Since these compounds have diverse chemical functionalities appended to the dithiocarbamate group, tuning of the surface energy of the modified surface is possible in concert with tuning of the work function.
Table 1 shows examples of dithiocarbamate comounds of Type I;
Table 2 shows examples of dithiocarbamate compounds of Type II;
Table 3 shows examples of dithiocarbamate compounds of Type III;
Table 4 shows examples of dithiocarbamate compounds of Type IV;
Table 5 shows examples of dithiocarbamate compounds of Type V;
Table 6 shows examples of dithiocarbamate compounds of Type VIa;
Table 7 shows examples of dithiocarbamate compounds of Types VIb and VIc;
Table 8 shows examples of dithiocarbamate compounds of Type VId;
Table 9 shows examples of dithiocarbamate compounds of Type VIe;
Table 10 shows examples of dithiocarbamate compounds of Type VII;
Table 11 shows examples of dithiocarbamate compounds of Type VIII;
Table 12 shows examples of dithiocarbamate compounds of Type IX;
Table 13 shows examples of dithiocarbamate compounds of Type Z-Ia and Z-Ib;
Table 14 shows examples of dithiocarbamate compounds of Type Z-Ic;
Table 15 shows examples of dithiocarbamate compounds of Type Z-II.

It should be noted, that in all the tables, the dithiocarbamate compounds are shown attached to a gold atom. Such gold atom, however, is not part of the structure of the dithiocarbamate compounds, but is part of the/a substrate.

Moreover, reference is made to the figures, wherein
**Figure 1** (prior art) illustrates the principle behind using self-assembled monolayers (SAMs) for (b) decreasing or (c) increasing the work function of metals at metal/organic interfaces, thereby controlling charge injection barriers (energy level alignment).
**Figure 2** (prior art) illustrates how a dipolar self-assembled monolayer increases or decreases the work function of the gate insulator of an organic thin-film transistor (OTFT) and thus influences band bending and doping in the organic semiconductor (Appl. Phys. Lett. 90, 132104 (2007)).
**Figure 3** (prior art) illustrates how a dipolar self-assembled monolayer was used to modify the work function of a silver anode of a top-emitting organic light-emitting diode (TOLED) (Langmuir 23, 7090 (2007)).
**Figure 4** (prior art) illustrates how a dipolar self-assembled monolayer was used to increase the work function of gold source/drain electrodes of an OTFT and thus reduce the Schottky barrier between the electrodes and the organic semiconductor (C₆₀) (Appl. Phys. Lett. 94, 083310 (2009)).
**Figure 5** (prior art) illustrates how a dipolar self-assembled monolayer was used to modify the performance of a solid-state photovoltaic solar cell (Adv. Mater. 20, 2376 (2008)).
**Figure 6** (prior art) illustrates how a dipolar self-assembled monolayer was used to modify the energy of the conduction band (*E*_{CB}) of titanium dioxide with respect to the redox potential (*E*_{redox}) of the electrolyte in a dye-sensitized solar cell (DSSC), thereby either (b) decreasing or (c) increasing the open-circuit voltage (V_{oc}) (J. Phys. Chem. B 109, 18907 (2005)).
**Figure 7** shows ultraviolet photoelectron spectra of monolayers of dimethyldithiocarbamate and n-butanethiol on gold in the energy range including the ionization threshold.
**Figure 8** shows ultraviolet photoelectron spectra of monolayers of eight dithiocarbamate compounds on gold in the energy range including the ionization threshold.
**Figure 9** shows the calculated (DFT) partial charge distributions and associated dipole moment for dimethyldithiocarbamate attached to a gold atom, and dipole moments of two piperazine dithiocarbamate compounds attached to gold atoms. Gold atoms are attached to the sulfur atoms of the dithiocarbamate groups for calculation purposes to simulate a gold surface and are not part of the dithiocarbamate compounds in accordance with the present invention.
**Figure 10** is a graph of the work function values of gold substrates modified with monolayers of various dithiocarbamate compounds versus the calculated molecular dipole moments. The line drawn is the linear fit to the data.
**Figure 11** is a graph of the work function values of silver and gold substrates modified with monolayers of five dithiocarbamate compounds versus the calculated molecular dipole moments. The solid line is the linear fit to the data for silver and the dashed line for gold has the same slope and intercept as the line in Figure 10.
**Figure 12** schematically illustrates three preferred dithiocarbamate compounds of the present invention with three kinds of rod-like segments attached to the dithiocarbamate group: a non-polar segment, a polar segment whose dipole moment has the same direction as that of the dithiocarbamate group, and a polar segment whose dipole moment has the opposite direction as that of the dithiocarbamate group.
**Figure 13** schematically illustrates three preferred embodiments of the present invention utilizing self-assembled monolayers comprising vertically oriented dithiocarbamate compounds with either a non-polar rod-like segment or a polar rod-like segment attached to the dithiocarbamate group, wherein the dipole moment of the polar segment has either the same or opposite direction as the dipole moment (not shown) of the dithiocarbamate group.
**Figure 14** schematically illustrates two preferred embodiments of the present invention utilizing self-assembled monolayers comprising tilted (θ = 45°) dithiocarbamate compounds with polar rod-like segments attached to the dithiocarbamate group, wherein the dipole moment of the polar segment has either the same or opposite direction as the dipole moment (not shown) of the dithiocarbamate group.
**Figure 15** schematically illustrates two preferred embodiments of the present invention utilizing a mixed self-assembled monolayer comprising two kinds of vertically oriented dithiocarbamate compounds, one with a polar rod-like segment attached to the dithiocarbamate group and one with a less polar rod-like segment attached to the dithiocarbamate group, wherein the dipole moment of the polar segment has either the same or opposite direction as the dipole moment (not shown) of the dithiocarbamate group.
**Figure 16** schematically illustrates two preferred embodiments of the present invention utilizing a self-assembled monolayer comprising a vertically oriented dithiocarbamate compound with a non-polar rod-like segment, wherein said segment is chemically converted into a polar rod-like segment, the dipole moment of said polar segment having either the same or opposite direction as the dipole moment (not shown) of the dithiocarbamate group.
**Figure 17** schematically illustrates a preferred embodiment of the present invention utilizing a self-assembled monolayer comprising a dipolar dithiocarbamate compound in a light-emitting diode.
**Figure 18** schematically illustrates a preferred embodiment of the present invention utilizing a self-assembled monolayer comprising a dipolar dithiocarbamate compound in a light-emitting diode.
**Figure 19** schematically illustrates a preferred embodiment of the present invention utilizing a self-assembled monolayer comprising a dipolar dithiocarbamate compound in a field-effect transistor or thin film transistor.
**Figure 20** schematically illustrates a preferred embodiment of the present invention utilizing a self-assembled monolayer comprising a dipolar dithiocarbamate compound in a field-effect transistor or thin film transistor.
**Figure 21** schematically illustrates a preferred embodiment of the present invention utilizing a self-assembled monolayer comprising a dipolar dithiocarbamate compound in a field-effect transistor or thin film transistor.
**Figure 22** schematically illustrates a preferred embodiment of the present invention utilizing self-assembled monolayers comprising a dipolar dithiocarbamate compound in a diode or rectifier.
**Figure 23** schematically illustrates a preferred embodiment of the present invention utilizing self-assembled monolayers comprising a dipolar dithiocarbamate compound in a diode or rectifier.
**Figure 24** provides an Example of implementation of the embodiment shown in Figure 22.
**Figure 25** provides another Example of implementation of the embodiment shown in Figure 22.

### Examples

**Table 1: Examples of dithiocarbamate compounds of Type I**

| **Type** | **Structure** | µ **(D)** | µ**ₓ (D)** | ΔΦ |
|---|---|---|---|---|
| Ib | 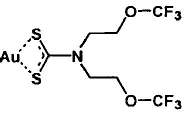 | -0.64 | -0.64 | - |
| Ib | 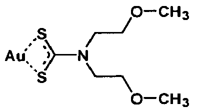 | +2.89 | +2.89 | - |
| Ia | 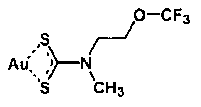 | +2.97 | +1.43 | - |
| Ib | 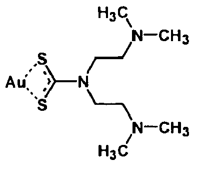 | +3.97 | +3.86 | - |
| Ib | 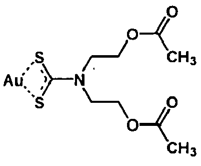 | +4.75 | +0.48 | - |

**Table 2: Examples of dithiocarbamate compounds of Type II**

| **Type** | **Structure** | µ**(D)** | µ**ₓ (D)** | ΔΦ |
|---|---|---|---|---|
| IIb | 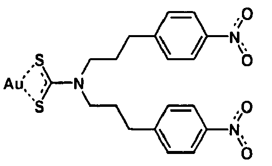 | -7.73 | -7.73 | + |
| IIa | 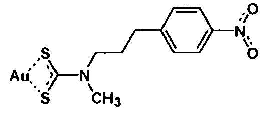 | -6.25 | -2.44 | + |
| IIb | 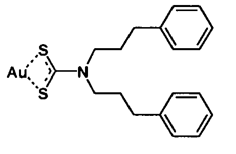 | +4.09 | +4.09 | - |
| IIa | 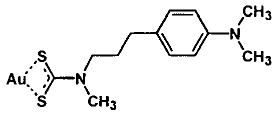 | +7.22 | +7.12 | - |
| IIb | 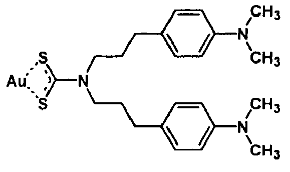 | +10.35 | +10.30 | - |

**Table 3: Examples of dithiocarbamate compounds of Type III**

| **Type** | **Structure** | µ**(D)** | µ**x(D)** | ΔΦ |
|---|---|---|---|---|
| IIIa | 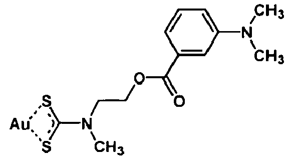 | +7.53 | +7.24 | - |
| IIIa | 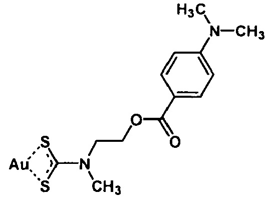 | +7.55 | +5.63 | - |
| IIIa | 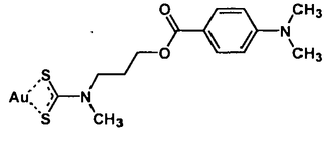 | +8.12 | +7.44 | - |
| IIIa | 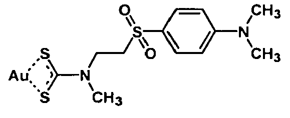 | +8.97 | +6.51 | - |
| IIIb | 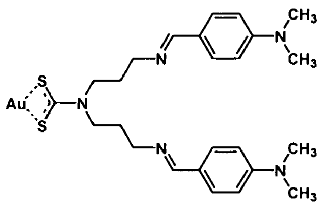 | +12.07 | +11.04 | - |
| IIIb | 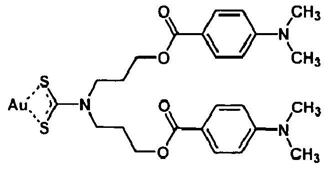 | +12.31 | +12.09 | - |
| IIIb | 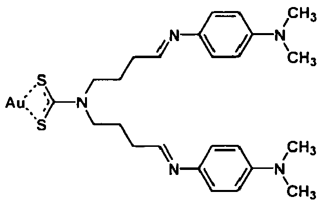 | +12.54 | +12.16 | - |
| IIIb | 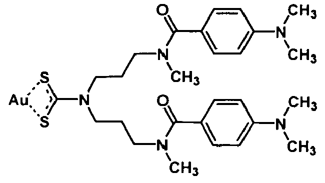 | +12.72 | +12.04 | - |
| IIIb | 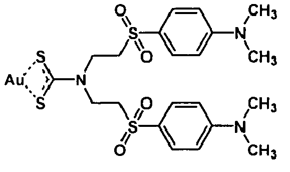 | +12.75 | +11.53 | - |
| IIIb | 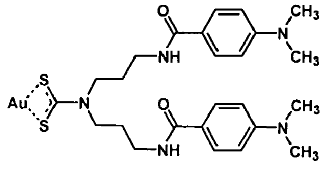 | +14.10 | +12.57 | - |

**Table 4: Examples of dithiocarbamate compounds of Type IV**

| **Type** | **Structure** | µ**(D)** | µ**ₓ(D)** | ΔΦ |
|---|---|---|---|---|
| IVb | 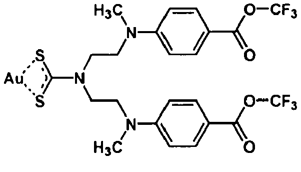 | -11.67 | -11.63 | + |
| IVb | 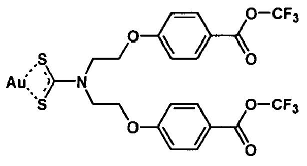 | -9.92 | -9.24 | + |
| IVb | 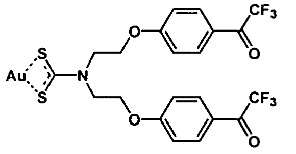 | -8.48 | -8.41 | + |
| IVb | 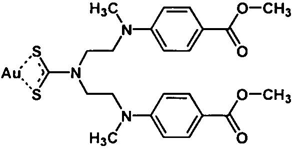 | -3.74 | -1.79 | + |
| IVa | 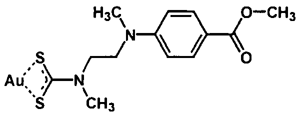 | +1.95 | +0.39 | - |
| IVb | 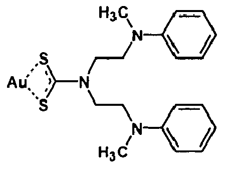 | +2.37 | +2.36 | - |
| IVb | 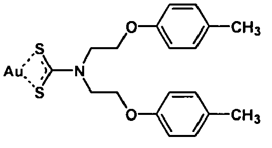 | +3.28 | +3.28 | - |
| IVb | 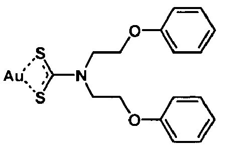 | +3.41 | +3.41 | - |
| IVb | 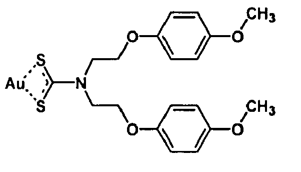 | +4.90 | +4.45 | - |

**Table 5: Examples of dithiocarbamate compounds of Type V**

| **Type** | **Structure** | µ**(D)** | µ**ₓ(D)** | ΔΦ |
|---|---|---|---|---|
| Ve | 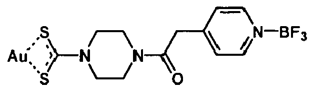 | -9.74 | -9.31 | + |
| Ve | 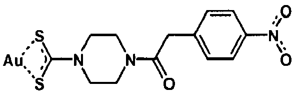 | -6.73 | -6.41 | + |
| Vf | 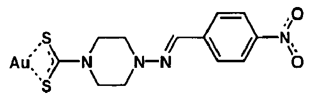 | -6.68 | -6.56 | + |
| Vb | 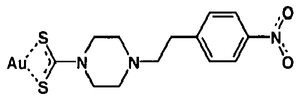 | -4.93 | -4.37 | + |
| Vd | 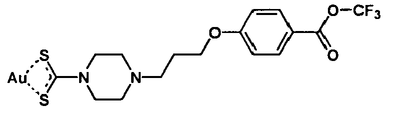 | -4.05 | -3.48 | + |
| Ve | 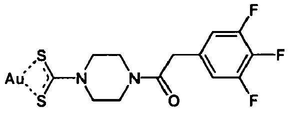 | -3.61 | -2.70 | + |
| Ve | 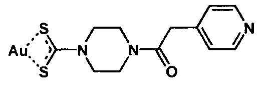 | -2.97 | -1.46 | + |
| Va | 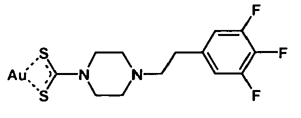 | +1.70 | +0.49 | - |
| Va | 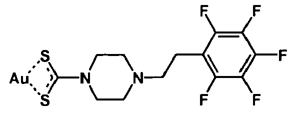 | +1.90 | +1.53 | - |
| Ve | 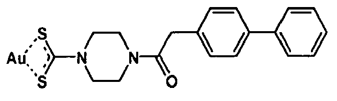 | +2.94 | +1.66 | - |
| Va | 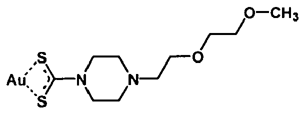 | +3.58 | +3.58 | - |
| Va | 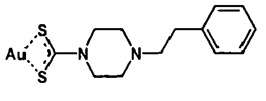 | +4.19 | +4.16 | - |
| Va | 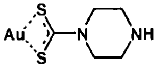 | +4.25 | +4.20 | - |
| Va | 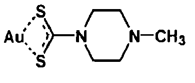 | +4.25 | +4.25 | - |
| Va | 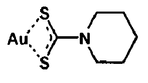 | +4.37 | +4.36 | - |
| Va | 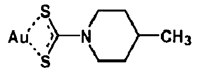 | +4.44 | +4.42 | - |
| Va | 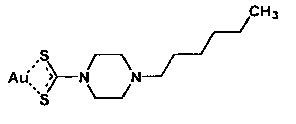 | +4.70 | +4.69 | - |
| Va | 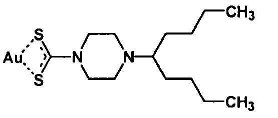 | +4.98 | +4.89 | - |
| Vb | 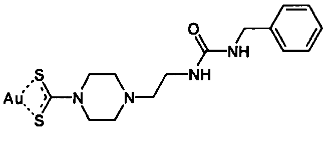 | +6.21 | +5.63 | - |
| Vf | 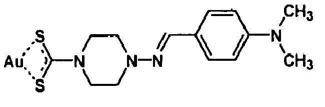 | +6.38 | +6.19 | - |
| Vd | 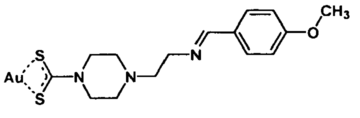 | +6.84 | +6.22 | - |
| Vc | 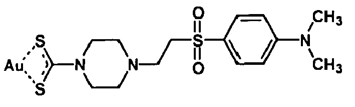 | +7.31 | +5.60 | - |
| Ve | 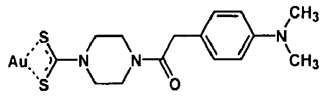 | +7.56 | +7.05 | - |
| Vg | 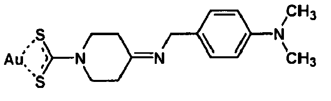 | +7.98 | +7.70 | - |
| Vb | 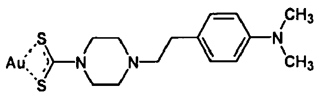 | +8.89 | +8.85 | - |
| Ve | 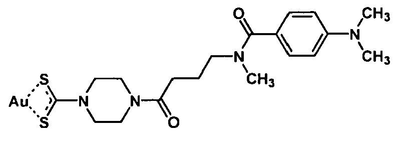 | +9.23 | +8.97 | - |
| Vc | 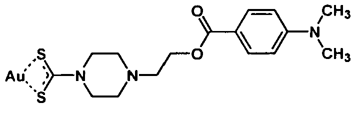 | +9.48 | +9.43 | - |
| Ve | 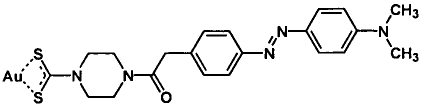 | +10.02 | +9.58 | - |
| Vd | 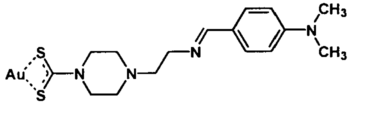 | +10.09 | +9.89 | - |
| Ve | 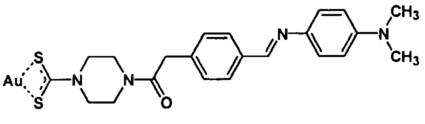 | +10.22 | +9.88 | - |
| Vd | 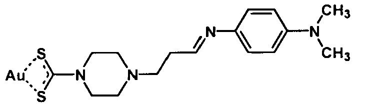 | +10.23 | +10.09 | - |
| Ve | 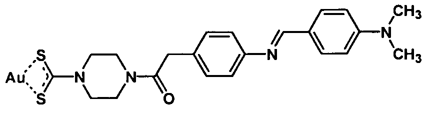 | +10.92 | +10.08 | - |
| Vg | 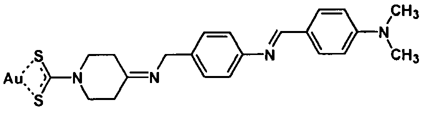 | +11.17 | +10.59 | - |
| Vg | 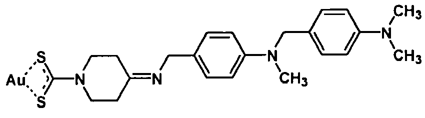 | +11.56 | +11.21 | - |

**Table 6: Examples of dithiocarbamate compounds of Type VIa**

| **Type** | **Structure** | µ**(D)** | µ**ₓ(D)** | ΔΦ |
|---|---|---|---|---|
| VIa | 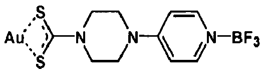 | -8.91 | -8.88 | + |
| VIa | 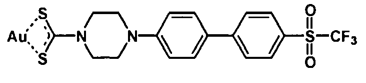 | -5.58 | -5.52 | + |
| VIa | 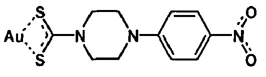 | -4.97 | -4.94 | + |
| VIa | 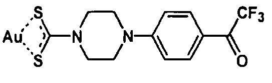 | -4.45 | -4.36 | + |
| VIa | 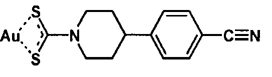 | -3.96 | -1.81 | + |
| VIa | 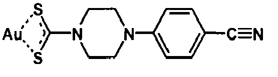 | -3.45 | -3.42 | + |
| VIa | 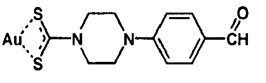 | -3.32 | -2.67 | + |
| VIa | 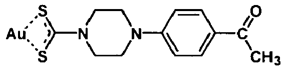 | -2.15 | -0.13 | + |
| VIa | 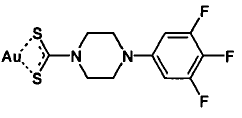 | -1.48 | -0.83 | + |
| VIa | 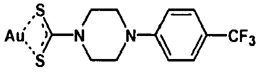 | -1.29 | -1.14 | + |
| VIa | 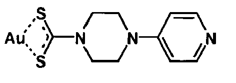 | -0.45 | -0.27 | + |
| VIa | 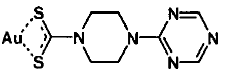 | +0.72 | +0.71 | - |
| VIa | 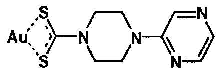 | +1.83 | +1.76 | - |
| VIa | 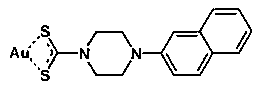 | +2.21 | +2.10 | - |
| VIa | 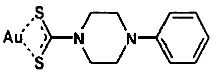 | +2.38 | +2.36 | - |
| VIa | 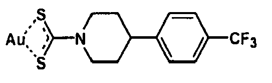 | +2.75 | +0.01 | - |
| VIa | 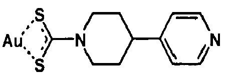 | +2.76 | +1.09 | - |
| VIa | 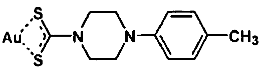 | +2.97 | +2.94 | - |
| VIa | 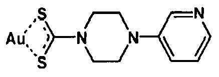 | +3.06 | +1.84 | - |
| VIa | 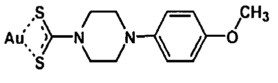 | +3.48 | +3.39 | - |
| VIa | 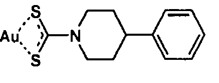 | +3.49 | +3.18 | - |
| VIa | 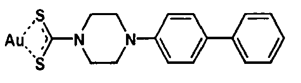 | +3.67 | +3.57 | - |
| VIa | 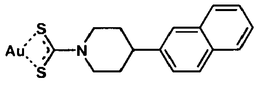 | +3.79 | +3.65 | - |
| VIa | 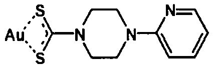 | +4.29 | +3.98 | - |
| VIa | 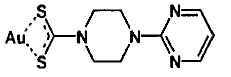 | +4.29 | +4.28 | - |
| VIa | 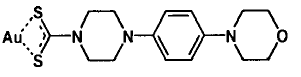 | +5.61 | +5.59 | - |
| VIa | 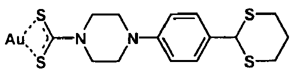 | +6.26 | +6.00 | - |
| VIa | 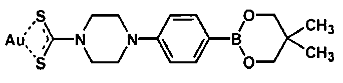 | +6.48 | +6.40 | - |
| VIa | 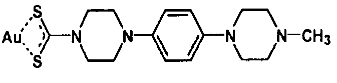 | +7.88 | +7.78 | - |
| VIa | 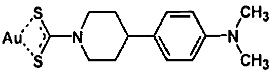 | +8.00 | +7.73 | - |
| VIa | 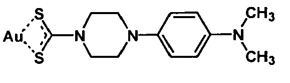 | +8.91 | +8.87 | - |
| VIa | 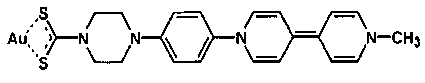 | +19.21 | +19.118 | - |

**Table 7: Examples of dithiocarbamate compounds of Types VIb and VIc**

| **Type** | **Structure** | µ**(D)** | µ**ₓ(D)** | ΔΦ |
|---|---|---|---|---|
| VIc | 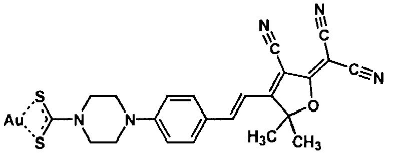 | -17.19 | -14.18 | + |
| VIc | 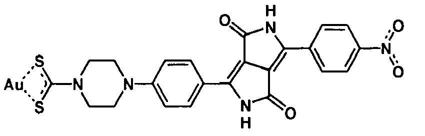 | -11.12 | -11.03 | + |
| VIc | 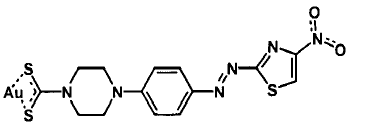 | -10.94 | -9.56 | + |
| VIc | 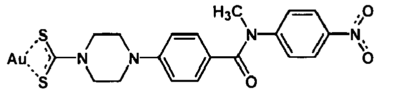 | -9.22 | -8.25 | + |
| VIc | 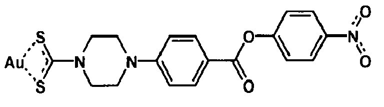 | -8.56 | -8.51 | + |
| VIc | 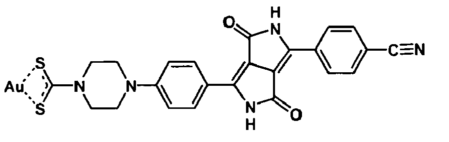 | -7.79 | -7.73 | + |
| VIc | 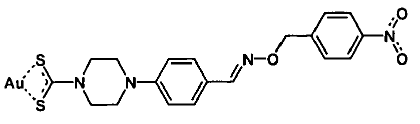 | -7.35 | -6.88 | + |
| VIc | 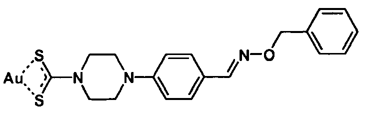 | +3.51 | +3.41 | - |
| VIc | 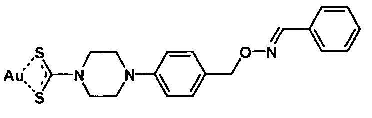 | +3.55 | +3.35 | - |
| VIc | 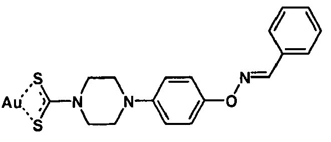 | +5.89 | +5.66 | - |
| VIc | 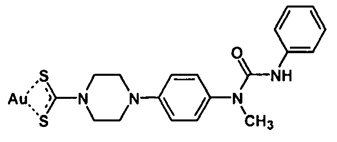 | +5.92 | +4.97 | - |
| VIc | 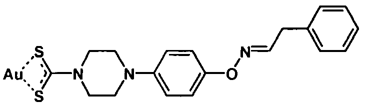 | +6.63 | +6.24 | - |
| VIc | 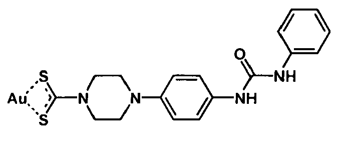 | +6.83 | +5.85 | - |
| VIb | 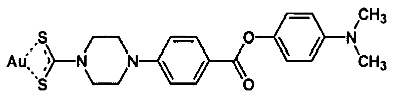 | +7.51 | +7.33 | - |
| VIc | 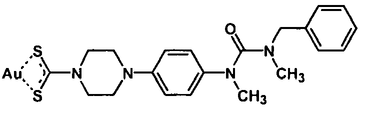 | +7.67 | +7.33 | - |
| VIc | 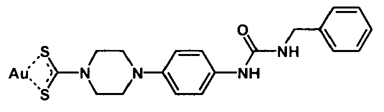 | +8.05 | +7.04 | - |
| VIb | 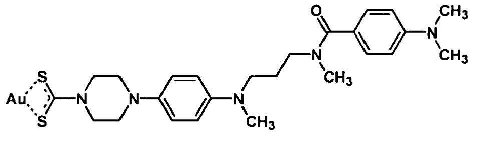 | +9.99 | +8.58 | - |
| VIb | 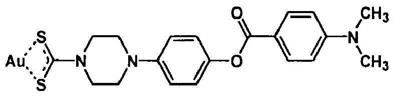 | +10.16 | +9.95 | - |
| VIb | 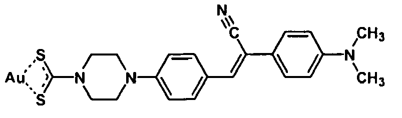 | +10.20 | +9.62 | - |
| VIb | 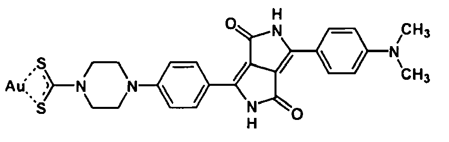 | +10.51 | +10.48 | - |
| VIb | 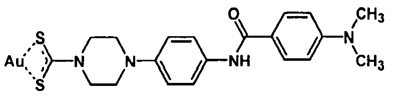 | +11.15 | +10.88 | - |
| VIb | 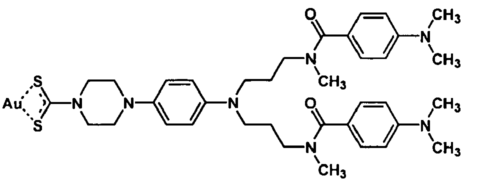 | +11.76 | +11.28 | - |
| VIb | 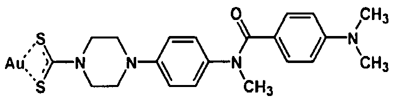 | +11.89 | +11.59 | - |
| VIb | 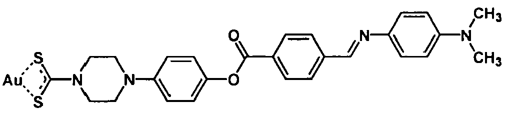 | +12.62 | +12.32 | - |
| VIb | 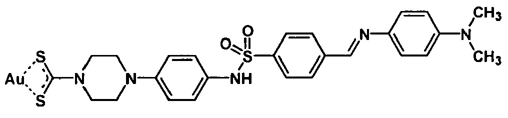 | +13.07 | +12.89 | - |
| VIb | 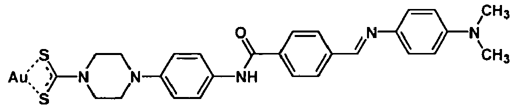 | +13.11 | +12.80 | - |
| VIb | 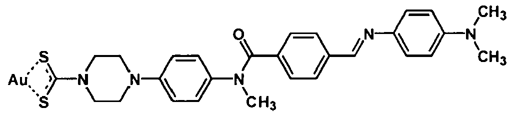 | +13.35 | +12.83 | - |
| VIb | 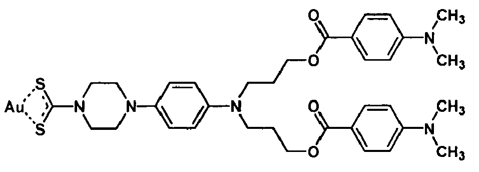 | +13.90 | +13.81 | - |
| VIb | 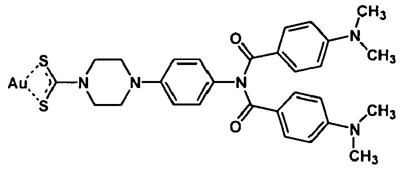 | +15.54 | +15.53 | - |
| VIb | 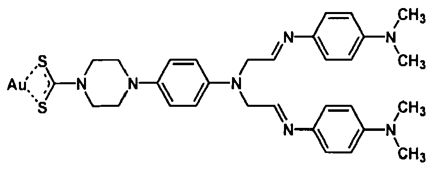 | +15.87 | +15.78 | - |

**Table 8: Examples of dithiocarbamate compounds of Type VId**

| **Type** | **Structure** | µ**(D)** | µ**ₓ(D)** | ΔΦ |
|---|---|---|---|---|
| VId | 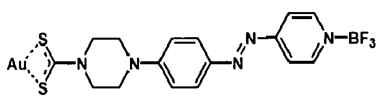 | -15.81 | -15.76 | + |
| VId | 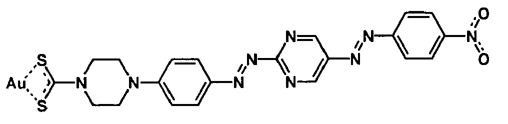 | -14.13 | -14.02 | + |
| VId | 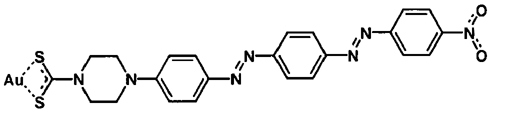 | -13.61 | -13.54 | + |
| VId | 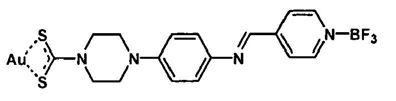 | -12.98 | -12.98 | + |
| VId | 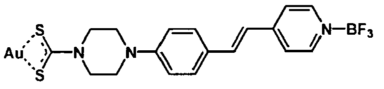 | -12.66 | -12.59 | + |
| VId | 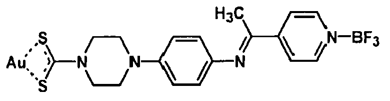 | -11.38 | -11.24 | + |
| VId | 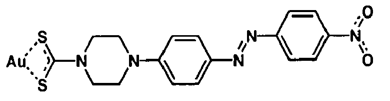 | -9.60 | -9.56 | + |
| VId | 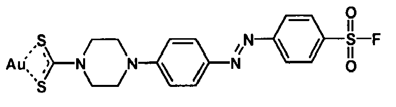 | -9.36 | -9.31 | + |
| VId | 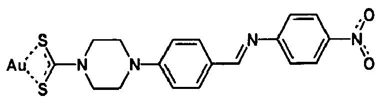 | -8.58 | -8.28 | + |
| VId | 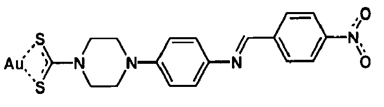 | -7.93 | -7.80 | + |
| VId | 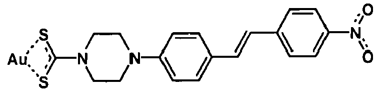 | -7.70 | -7.59 | + |
| VId | 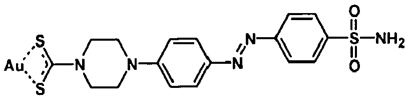 | -7.14 | -6.59 | + |
| VId | 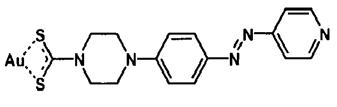 | -3.57 | -3.46 | + |
| VId | 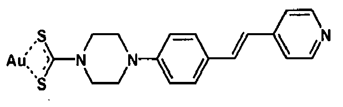 | -1.33 | -0.88 | + |
| VId | 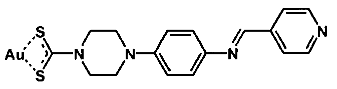 | -1.08 | -0.94 | + |
| VId | 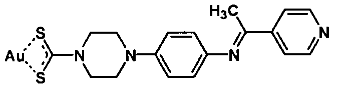 | +1.28 | +0.66 | - |
| VId | 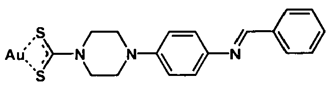 | +4.20 | +3.94 | - |
| VId | 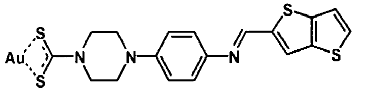 | +4.90 | +4.68 | - |
| VId | 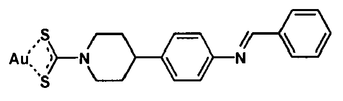 | +4.91 | +4.03 | - |
| VId | 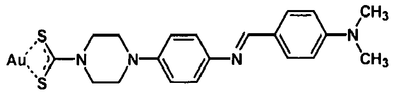 | +8.06 | +7.86 | - |
| VId | 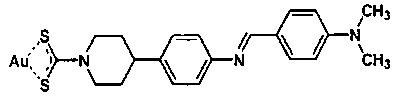 | +9.08 | +8.57 | - |
| VId | 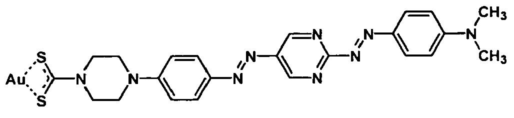 | +9.89 | +9.83 | - |
| VId | 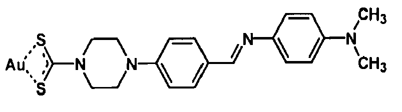 | +9.92 | +9.81 | - |
| VId | 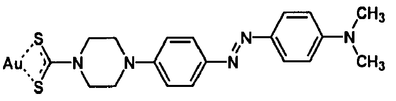 | +10.05 | +10.00 | - |
| VId | 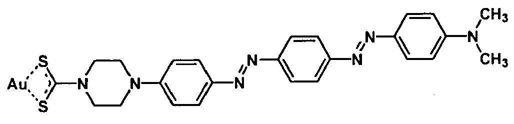 | +10.23 | +10.16 | - |
| VId | 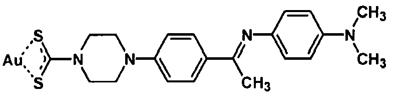 | +10.35 | +10.14 | - |
| VId | 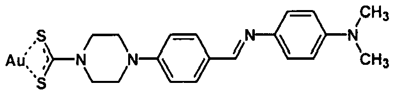 | +10.37 | +10.26 | - |
| VId | 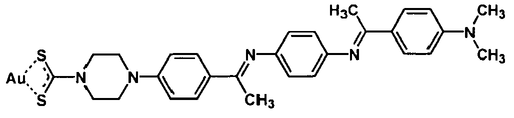 | +13.24 | +13.17 | - |
| VId | 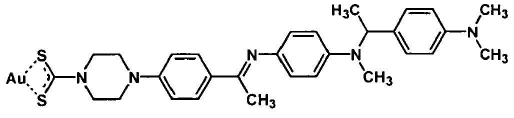 | +15.09 | +14.94 | - |

**Table 9: Examples of dithiocarbamate compounds of Type VIe**

| **Type** | **Structure** | µ**(D)** | µ**ₓ(D)** | ΔΦ |
|---|---|---|---|---|
| VIe | 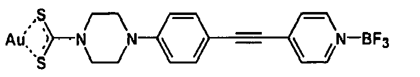 | -12.66 | -12.64 | + |
| VIe | 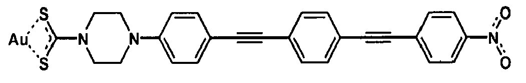 | -10.55 | -10.54 | + |
| VIe | 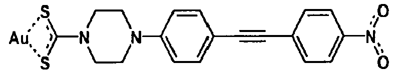 | -8.03 | -8.01 | + |
| VIe | 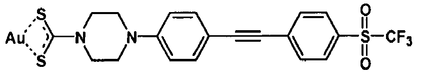 | -7.04 | -6.68 | + |
| VIe | 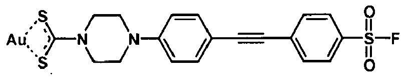 | -7.02 | -6.82 | + |
| VIe | 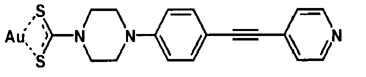 | -1.28 | -1.21 | + |
| VIe | 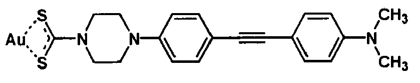 | +11.24 | +11.23 | - |
| VIe | 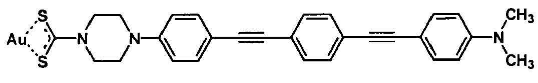 | +13.17 | +13.15 | - |

**Table 10: Examples of dithiocarbamate compounds of Type VII**

| **Type** | **Structure** | µ**(D)** | µ**ₓ(D)** | ΔΦ |
|---|---|---|---|---|
| VIIa | 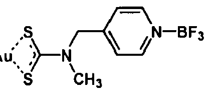 | -8.21 | -7.18 | + |
| VIIb | 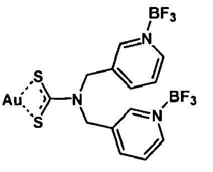 | -7.14 | -2.78 | + |
| VIIb | 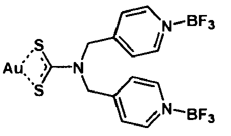 | -6.76 | -6.75 | + |
| VIIa | 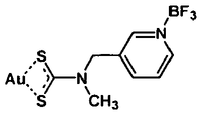 | -6.25 | -4.66 | + |
| VIIa | 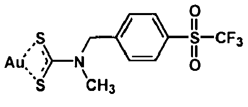 | -4.26 | -2.59 | + |
| VIIa | 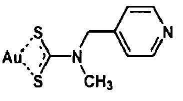 | -2.51 | -0.38 | + |
| VIIb | 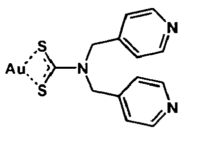 | +0.72 | +0.72 | - |
| VIIa | 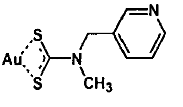 | +1.23 | +0.86 | - |
| VIIb | 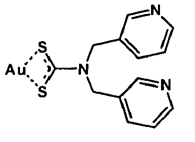 | +2.81 | +2.05 | - |
| VIIb | 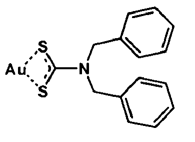 | +3.68 | +2.82 | - |
| VIIa | 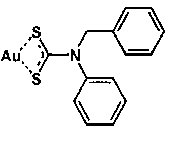 | +3.91 | +3.65 | - |
| VIIa | 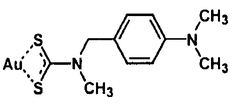 | +7.32 | +6.57 | - |

**Table 11: Examples of dithiocarbamate compounds of Type VIII**

| **Type** | **Structure** | µ**(D)** | µ**ₓ(D)** | ΔΦ |
|---|---|---|---|---|
| VIIIb | 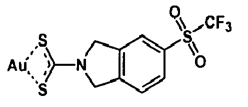 | -3.09 | -1.55 | + |
| VIIIa | 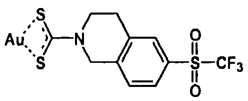 | -2.97 | -2.93 | + |
| VIIIb | 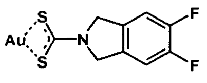 | +0.69 | +0.69 | - |
| VIIIb | 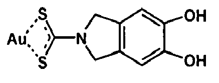 | +4.58 | +4.57 | - |
| VIIIb | 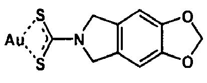 | +4.84 | +4.82 | - |
| VIIIb | 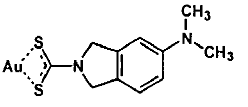 | +7.39 | +7.29 | - |
| VIIIa | 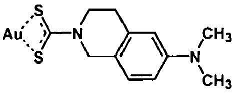 | +7.50 | +7.33 | - |

**Table 12: Examples of dithiocarbamate compounds of Type IX**

| **Type** | **Structure** | µ**(D)** | µ**ₓ(D)** | ΔΦ |
|---|---|---|---|---|
| IXb | 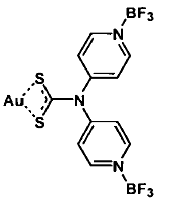 | -8.54 | -8.54 | + |
| IXa | 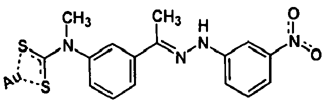 | -5.65 | -4.88 | + |
| IXa | 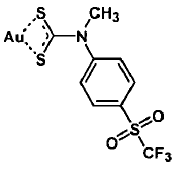 | -4.60 | -0.13 | + |
| IXb | 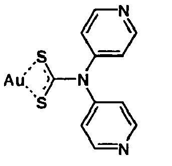 | +0.14 | +0.14 | - |
| IXb | 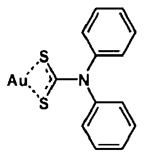 | +3.87 | +3.87 | - |
| IXa | 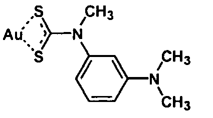 | +5.86 | +4.98 | - |
| IXa | 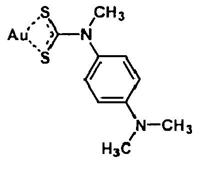 | +7.39 | +5.11 | - |

**Table 13: Examples of dithiocarbamate compounds of Type Z-Ia and Z-Ib**

| **Type** | **Structure** | µ**(D)** | µ**ₓ(D)** | ΔΦ |
|---|---|---|---|---|
| Z-Ia | 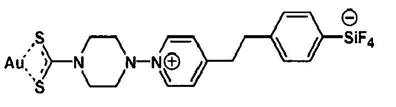 | -32.49 | -31.94 | + |
| Z-Ib | 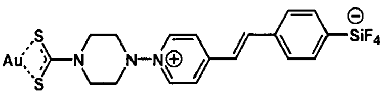 | -31.46 | -31.08 | + |
| Z-Ia | 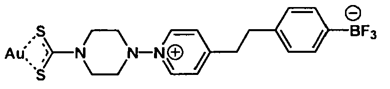 | -27.39 | -27.12 | + |
| Z-Ib | 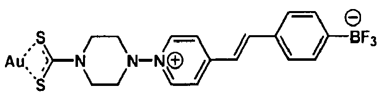 | -26.36 | -26.05 | + |
| Z-Ib | 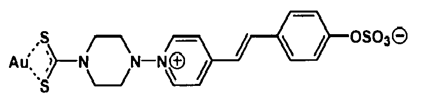 | -24.28 | -23.30 | + |
| Z-Ib | 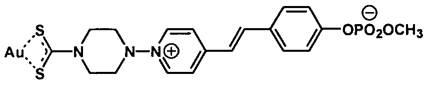 | -19.90 | -18.25 | + |
| Z-Ib | 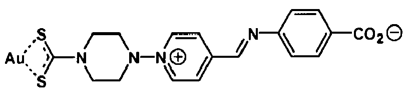 | -19.55 | -18.91 | + |
| Z-Ib | 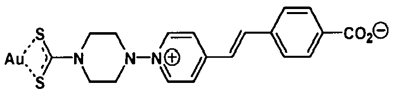 | -18.43 | -17.92 | + |

**Table 14: Examples of dithiocarbamate compounds of Type Z-Ic**

| **Type** | **Structure** | µ**(D)** | µ**ₓ(D)** | ΔΦ |
|---|---|---|---|---|
| Z-Ic | 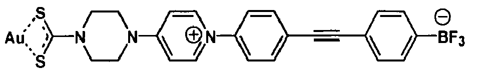 | -34.91 | -34.90 | + |
| Z-Ic | 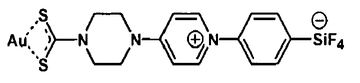 | -26.54 | -26.52 | + |
| Z-Ic | 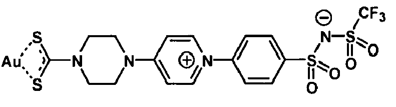 | -25.71 | -25.19 | + |
| Z-Ic | 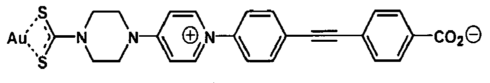 | -24.17 | -24.16 | + |
| Z-Ic | 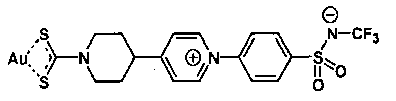 | -22.41 | -21.90 | + |
| Z-Ic | 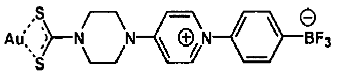 | -22.21 | -22.20 | + |
| Z-Ic | 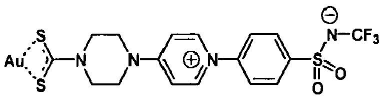 | -22.02 | -22.00 | + |
| Z-Ic | 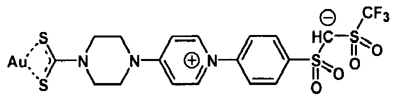 | -20.95 | -20.78 | + |
| Z-Ic | 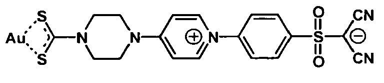 | -19.81 | -19.47 | + |
| Z-Ic | 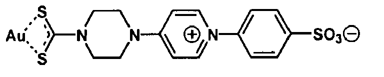 | -18.45 | -18.43 | + |
| Z-Ic | 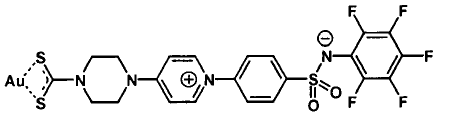 | -16.02 | -15.17 | + |
| Z-Ic | 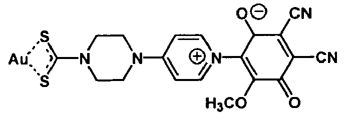 | -15.97 | -14.93 | + |
| Z-Ic | 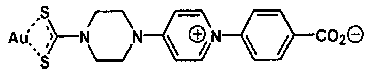 | -13.39 | -13.34 | + |
| Z-Ic | 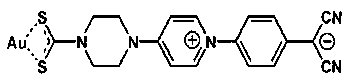 | -12.41 | -12.37 | + |
| Z-Ic | 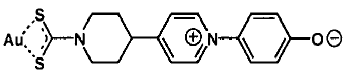 | -9.44 | -8.72 | + |
| Z-Ic | 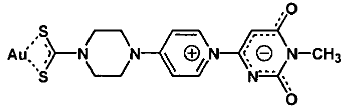 | -8.84 | -8.63 | + |
| Z-Ic | 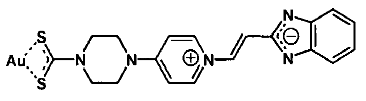 | -5.09 | -4.59 | + |
| Z-Ic | 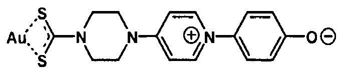 | -5.02 | -4.83 | + |
| Z-Ic | 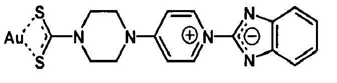 | +2.97 | +2.85 | - |
| Z-Ic | 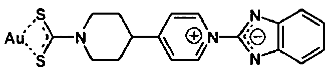 | +4.11 | +2.86 | - |

**Table 15: Examples of dithiocarbamate compounds of Type Z-II**

| **Type** | **Structure** | µ**(D)** | µ**ₓ(D)** | ΔΦ |
|---|---|---|---|---|
| Z-II | 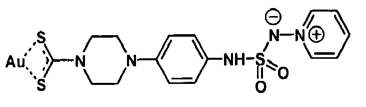 | +8.14 | +8.01 | - |
| Z-II | 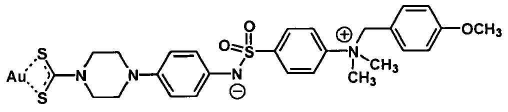 | +13.19 | +12.64 | - |
| Z-II | 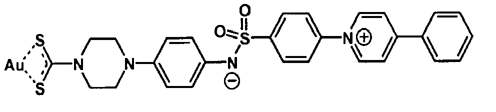 | +13.85 | +13.46 | - |
| Z-II | 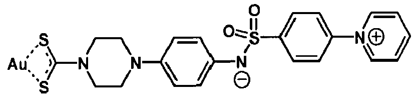 | +14.01 | +13.51 | - |
| Z-II | 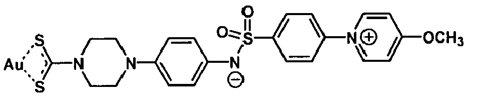 | +19.81 | +19.23 | - |
| Z-II | 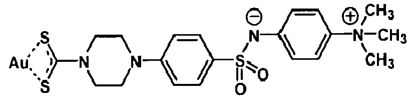 | +20.62 | +19.35 | - |
| Z-II | 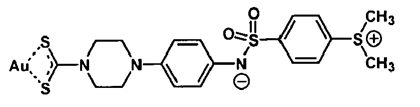 | +20.88 | +19.80 | - |
| Z-II | 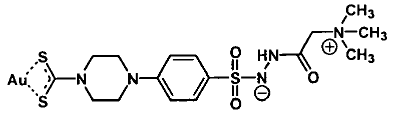 | +21.83 | +19.24 | - |
| Z-II | 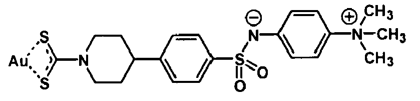 | +22.42 | +21.19 | - |
| Z-II | 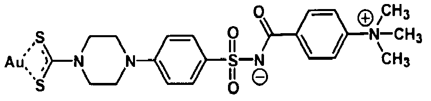 | +28.38 | +25.22 | - |
| Z-II | 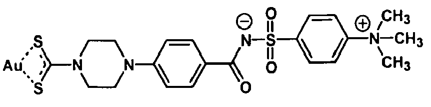 | +28.57 | +24.67 | - |
| Z-II | 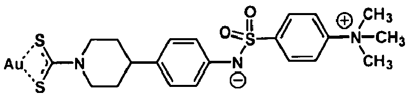 | +29.27 | +27.84 | - |
| Z-II | 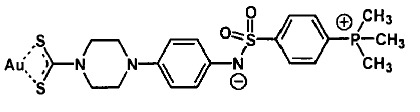 | +29.49 | +29.11 | - |
| Z-II | 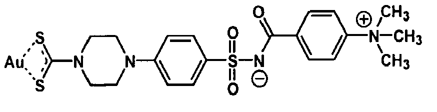 | +30.75 | +26.81 | - |
| Z-II | 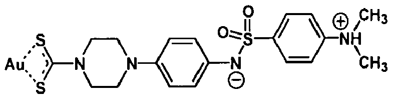 | +30.99 | +30.76 | - |
| Z-II | 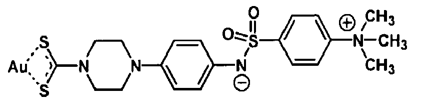 | +34.10 | +33.79 | - |
| Z-II | 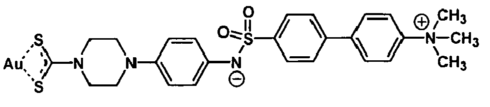 | +42.12 | +41.23 | - |

The features of the present invention disclosed in the specification, the claims and/or in the accompanying drawings, may, both separately, and in any combination thereof, be material for realizing the invention in various forms thereof.

## Claims

1. Use of a dithiocarbamate compound for modifying the work function of a conducting, semiconducting, or insulating inorganic substrate.

2. Use according to claim 1, wherein said use comprises the step:
Depositing a monolayer of said dithiocarbamate compound on a surface of said conducting, semiconducting, or insulating inorganic substrate.

3. Use according to any of claims 1-2, wherein said step of depositing occurs by exposing said conducting, semiconducting, or insulating inorganic substrate to a solution of a dithiocarbamate compound.

4. Use according to any of the foregoing claims, wherein said dithiocarbamate compound has a permanent positive or negative electrical dipole moment.

5. Use according to any of the foregoing claims, wherein said dithiocarbamate compound has an S₂CNH- group or an S₂CNR- group in its molecular structure, wherein R denotes an alkyl, aryl, aralkyl, heteroalkyl, heteroaryl or heteroaralkyl substituent, which itself is substituted or unsubstituted.

6. Use according to any of the foregoing claims, wherein said dithiocarbamate compound is a piperazine dithiocarbamate derivative or a piperidine dithiocarbamate derivative.

7. Use according to any of the foregoing claims, wherein said dithiocarbamate compound has at least one or several uncharged polar components in its molecular structure.

8. Use according to claim 7, wherein said dithiocarbamate compound has a dithiocarbamate group in its molecular structure having a dipole moment with a first polarity, and wherein said at least one or several uncharged polar components has a second polarity which is opposite said first polarity, wherein said dithiocarbamate compound is used for increasing the work function of said conducting, semiconducting, or insulating inorganic substrate.

9. Use according to claim 7, wherein said dithiocarbamate compound has a dithiocarbamate group in its molecular structure having a dipole moment with a first polarity, and wherein said at least one or several uncharged polar components has a second polarity which is the same as said first polarity, wherein said dithiocarbamate compound is used for decreasing the work function of said conducting, semiconducting, or insulating inorganic substrate.

10. Use according to any of the foregoing claims, wherein said dithiocarbamate compound is selected from the group having the general structures: 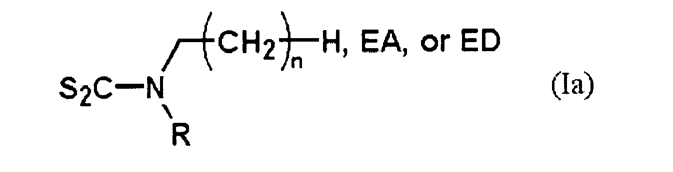 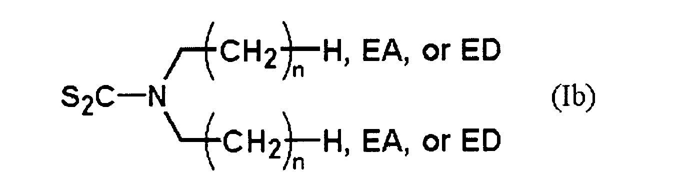 n=0-17
R = H, CH₃ or CH₂CH₃ ,
wherein H denotes an H-atom, EA denotes an electron-accepting group, and ED denotes an electron-donating group, 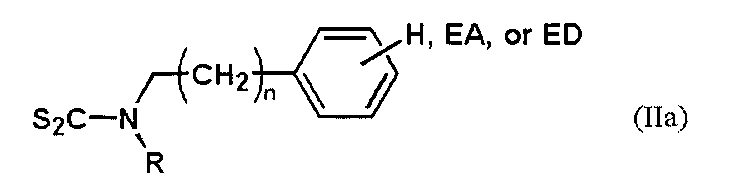 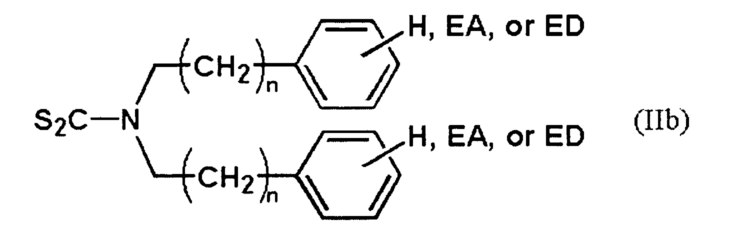 n=0-17
R = H, CH₃ or CH₂CH₃
wherein H denotes an H-atom, EA denotes an electron-accepting group, and ED denotes an electron-donating group, 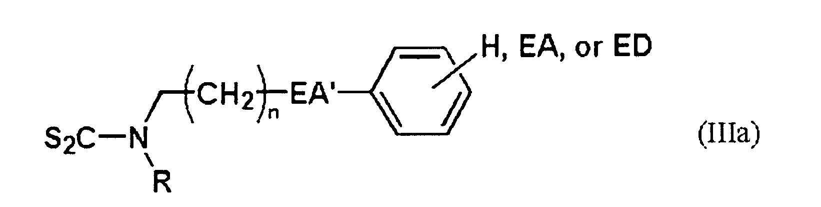 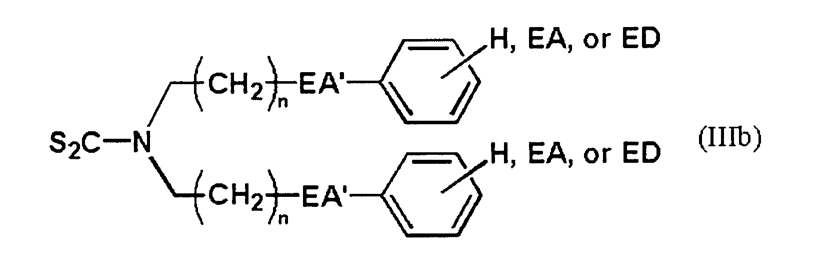 n=0-15
R = H, CH₃ or CH₂CH₃
wherein EA' denotes a bridging electron-accepting group selected from the group comprising -OC(O)-, -C(O)O-, -C(O)-, -S(O)₂-, -N(R')C(O)-, and -N(R')S(O)₂-, where R' = H or CH₃, and wherein H denotes an H-atom, EA denotes an electron-accepting group, and ED denotes an electron-donating group, 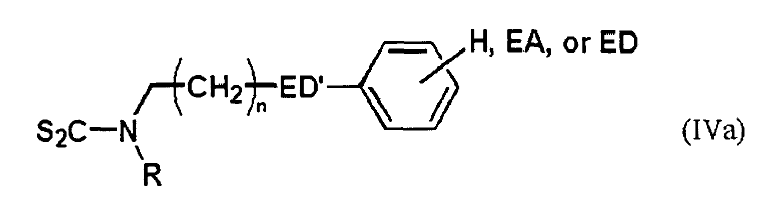 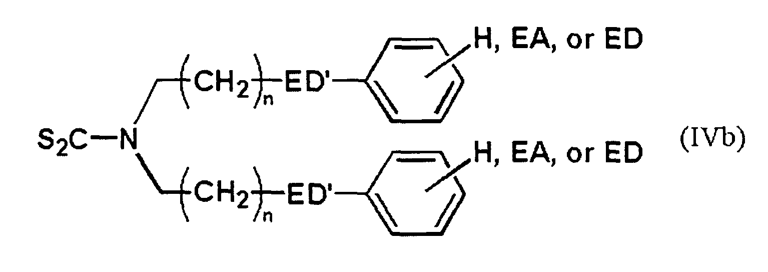 n=0-15
R = H, CH₃ or CH₂CH₃
wherein ED' denotes a bridging electron-donating group selected from the group comprising -N(R')-, -O-, -S-, and -C(O)N(R')-, where R' = H or CH₃, and wherein H denotes an H-atom, EA denotes an electron-accepting group, and ED denotes an electron-donating group, 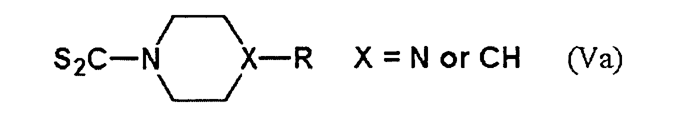
-R =
-H 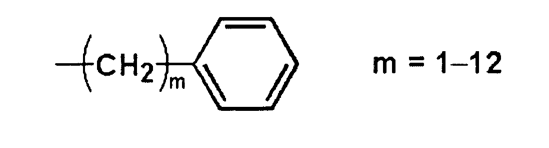 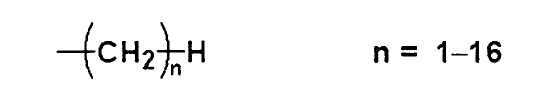 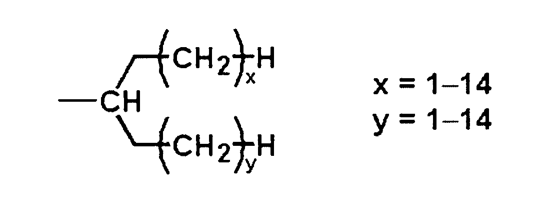 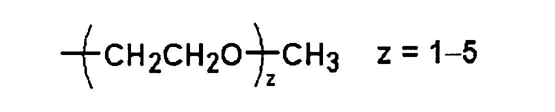
piperazine (X = N) or piperidine (X = CH) derivatives having the general structures: 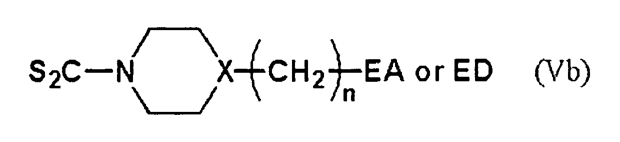 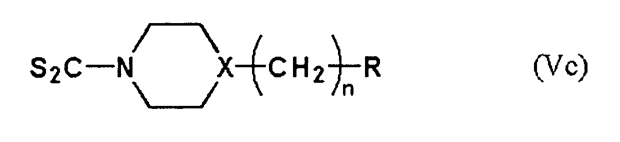 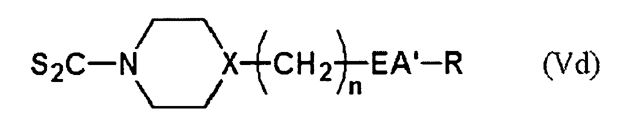 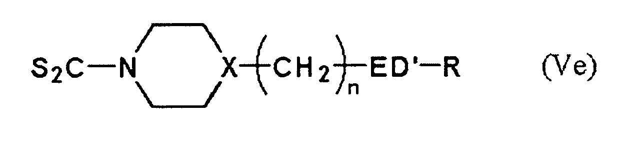 X=N orCH
n = 1-16 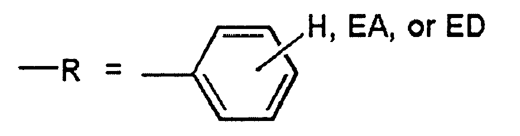 wherein EA' denotes a bridging electron-accepting group selected from the group comprising -OC(O)-, -C(O)O-, -C(O)-, and -S(O)₂-, wherein ED' denotes a bridging electron-donating group selected from the group comprising -N(R')-, -O-, and -S-, and wherein H denotes an H-atom, EA denotes an electron-accepting group, and ED denotes an electron-donating group,
piperazine or piperidine derivatives having the general structures: 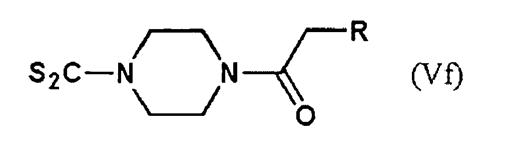 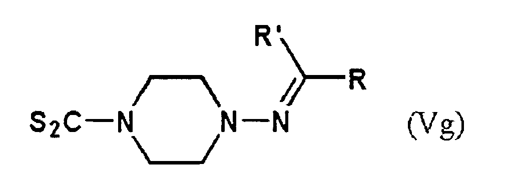 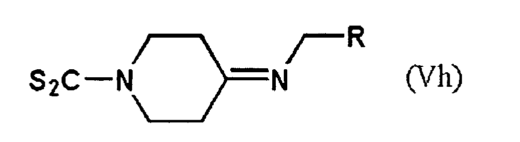 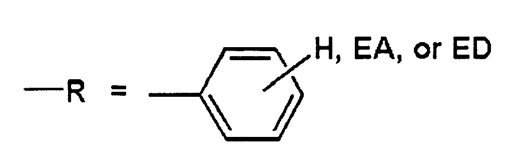 R' = H or CH₃
wherein H denotes an H-atom, EA denotes an electron-accepting group, and ED denotes an electron-donating group,
piperazine (X = N) or piperidine (X = CH) derivatives having the general structures: 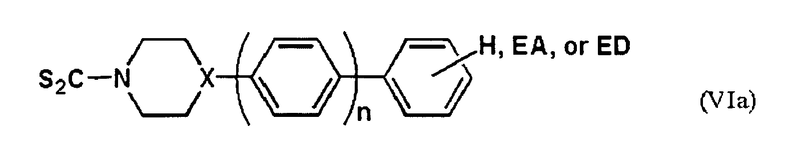 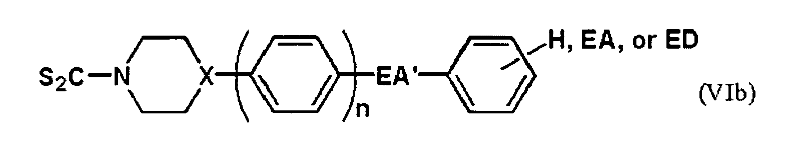 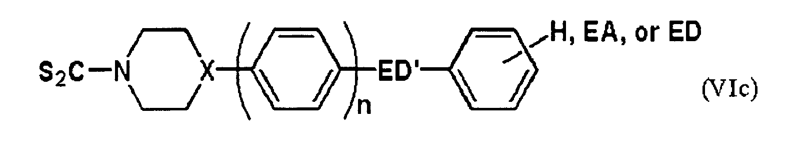 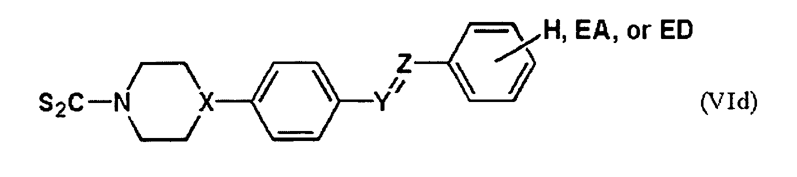 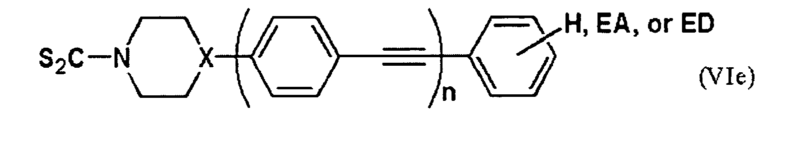 n=1-3
X=NorCH
Y = N, CH, or C(CH₃)
Z = N, CH, or C(CH₃)
wherein EA' denotes a bridging electron-accepting group selected from the group comprising -OC(O)-, -C(O)O-, -C(O)-, and -S(O)₂-, wherein ED' denotes a bridging electron-donating group selected from the group comprising -N(R')-, -O-, and -S-, and wherein H denotes an H-atom, EA denotes an electron-accepting group, and ED denotes an electron-donating group,
benzylamine derivatives having the general structures: 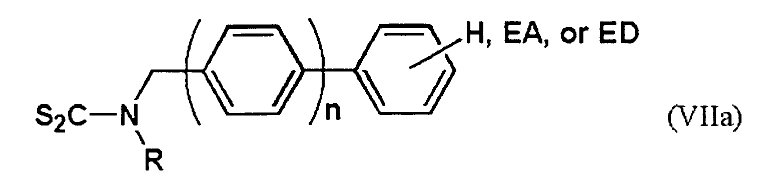 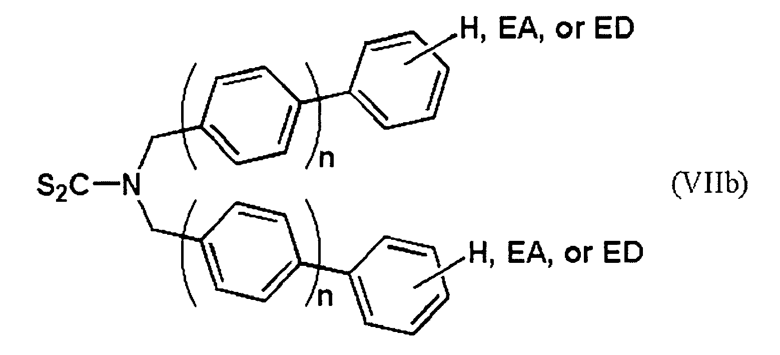 n=1-3
R = H, CH₃ or CH₂CH₃
wherein H denotes an H-atom, EA denotes an electron-accepting group, and ED denotes an electron-donating group,
tetrahydroisoquinoline or isoindoline derivatives having the general structures: 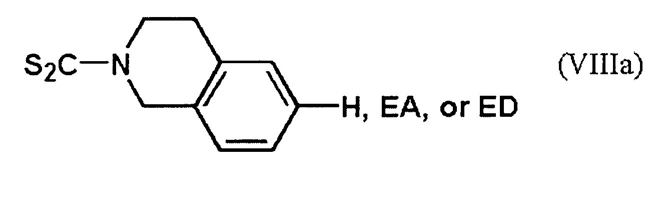 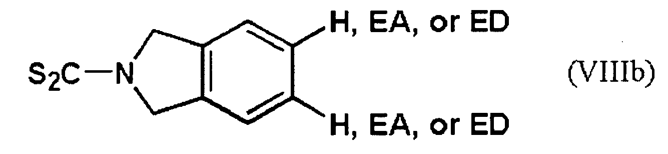 wherein H denotes an H-atom, EA denotes an electron-accepting group, and ED denotes an electron-donating group,
aniline or diphenylamine derivatives having the general structures: 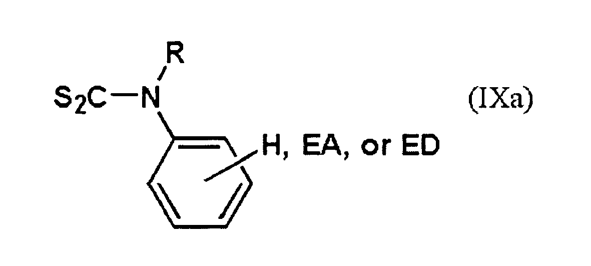 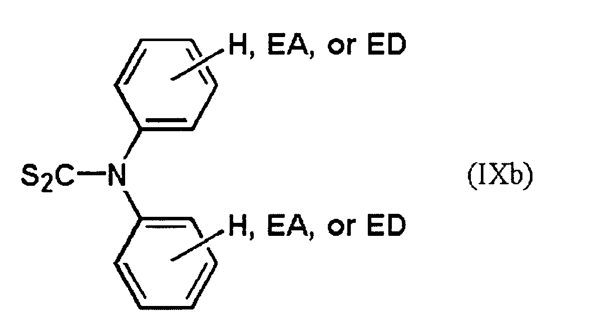 R = H, CH₃, CH₂CH₃, or CH₂C₆H₅
wherein H denotes an H-atom, EA denotes an electron-accepting group, and ED denotes an electron-donating group.

11. Use according to any of claims 1-6, wherein said dithiocarbonate compound is a zwitterionic dithiocarbonate compound.

12. Use according to claim 11, wherein said zwitterionic dithiocarbamate compound is selected from the group comprising piperazine (X = N) or piperidine (X = CH) derivatives having the general structures: 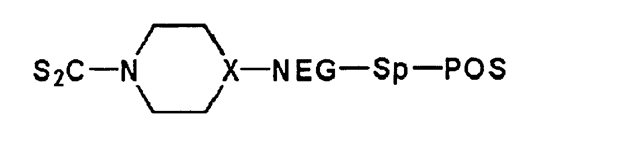 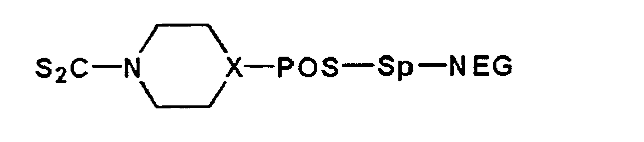 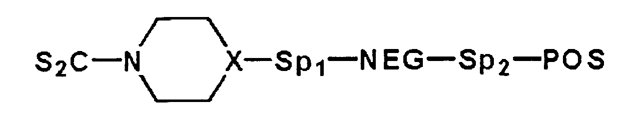 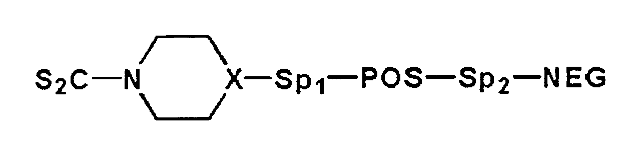 X=NorCH
wherein NEG denotes a negatively charged group, POS denotes a positively charged group, and Sp, Sp₁, and Sp₂ denote alkyl, aryl, or alkaryl connecting groups, or wherein said zwitterionic dithiocarbamate compound is selected from the group comprising piperazine (X = N) or piperidine (X = CH) derivatives having the general structures: 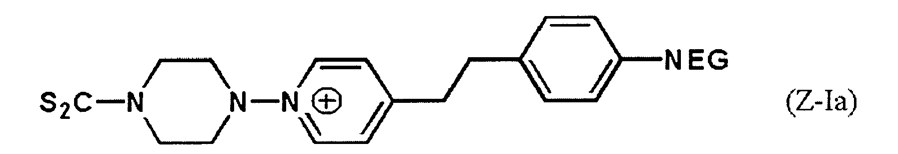 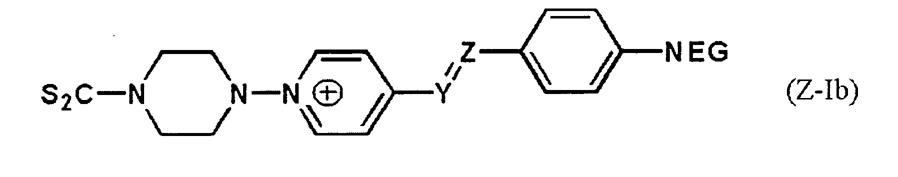 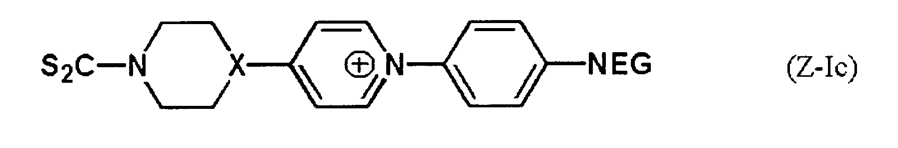 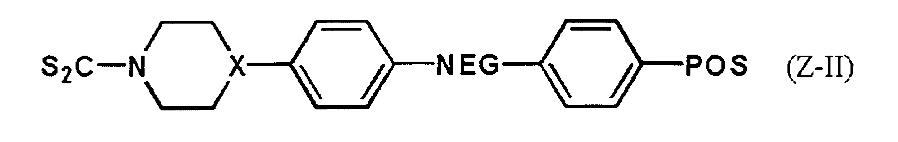 X = N or CH
Y = N, CH, or C(CH₃)
Z = N, CH, or C(CH₃) wherein NEG denotes a negatively charged group and POS denotes a positively charged group.

13. An assembly for use in an electronic device, said assembly comprising:
a) a conducting substrate, a semiconducting substrate, or an insulating inorganic substrate, said substrate having a surface,
b) a monolayer of at least one dithiocarbamate compound on said surface, wherein said monolayer is covalently bonded to said surface via an S₂CNH- or S₂CNR- group and is not in contact with another monolayer of at least one dithiocarbamate compound on the side opposite said surface, and wherein said dithiocarbamate compound is as defined in any of claims 4-12, and
c) an organic layer, an inorganic layer, or an electrolyte layer deposited on said monolayer.

14. An electronic device comprising the assembly according to claim 13, wherein, preferably, said device is selected from a light-emitting device, a Schottky barrier diode, a rectifier, a field effect transistor, a photovoltaic device, a photochemical device, a memory device, a sensing device, or a display.

15. A method of modifying the work function of a conducting, semiconducting, or insulating inorganic substrate, said method comprising the steps:
a) depositing a monolayer of said dithiocarbamate compound on a surface of said conducting, semiconducting, or insulating inorganic substrate, said dithiocarbamate compound, said substrate, said depositing step being as defined in any of claims 1-14, and
b) depositing an organic layer, an inorganic layer, or an electrolyte layer on said monolayer.
